# EUROPEAN PATENT APPLICATION

(11) **EP 4 174 491 A1**
(43) Date of publication of application: **03.05.2023**
(21) Application number: 22176234.7
(22) Date of filing: 30.05.2022
(51) Int. Cl.: G01N 33/52, C12Q 1/48, C09B 29/036, C12N 9/12, G01N 33/84

(54) **COMPOSITIONS AND METHODS FOR DETECTING PYROPHOSPHATE PRODUCTS OF ENZYME REACTIONS USING PYRIDYLAZOANILINE DYES**

(30) Priority: 01.11.2021 US 202163263361 P; 01.11.2021 US 202163263364 P; 04.05.2022 US 202263364115 P
(71) Applicant: NEW ENGLAND BIOLABS, INC., Ipswich, MA 01938-2723 (US)
(72) Inventor: TANNER, Nathan, Ipswich, 01938-2723 (US); CORREA, Ivan, Ipswich, 01938-2723 (US); ZHANG, Yinhua, Ipswich, 01938-2723 (US); ALPASLAN, Ece, Ipswich, 01938-2723 (US)
(74) Representative: Griffin, Philippa Jane

(57) **Abstract**

Provided herein is a composition comprising an enzyme that releases pyrophosphate from a substrate and a dye of Formula 1. A method for detecting pyrophosphate is also provided. A kit comprising a polymerase that releases pyrophosphate by hydrolysis of nucleoside triphosphates during nucleic acid replication, a divalent manganese salt, and the dye are also provided. The present composition, method and kits provide a way to detect and/or quantify substrates or products of enzyme reacted substrates associated with the release pyrophosphate (e.g., nucleic acid amplification reactions and other reactions that hydrolyze ATP) via a distinct color change without substantially affecting the sensitivity and/or specificity of the reaction.

## Description

### BACKGROUND

A surprising finding in 2012 by Zhang, et al. and Tanner, et al. was that amplification of a target DNA could be detected in a low concentration buffer by a visual color change of a pH sensitive dye (see US 9,034,606, US 9,580,148 and US 11,162,133). The color change caused by a change in pH was the result of release of hydrogen ions (H⁺) from deoxyribose nucleoside triphosphates (dNTPs) when they were incorporated into DNA during amplification. The selected dyes did not appear to adversely affect the activity of the polymerase required for amplification; the amplification conditions themselves did not adversely affect a change in color; observable color change occurred as a direct result of amplification; the color change of the dye was readily detectable by eye; and the detection means was sensitive enough to detect low amounts of amplified DNA product where the relatively low concentrations of DNA were either due to the concentration of the starting material or to the number of amplification cycles required to reach a detectable end point.

Previous to these findings, color dyes such as calcein or hydroxynaphthol blue (HNB) were added to an amplification reaction but gave color changes that was ambiguous at best. Goto, et al., Biotechniques, 46, 3, 167-172, 2009, described the use of HNB as a colorimetric reagent for alkaline earth metal ions in loop-mediated isothermal amplification (LAMP) reactions. The color of HNB was pH dependent and was magenta at pH 8.6- pH 9.0 and violet at pH 8.4. Alternatively, color change was induced by a change in magnesium ion concentration irrespective of pH. The maximum absorbance peak was observed at 650 nm in the absence of magnesium with the maximum absorbance peak decreasing as the level of magnesium increased. Because of the ambiguity of the HNB color change visualized by eye resulting from amplification, it was necessary to use some other method of detecting the occurrence of LAMP.

Alternatively, color dyes were added after amplification had occurred to detect DNA amplicons. The dyes would bind to DNA either directly or by intercalation so that the more DNA product that was made, the more the intensity of the color from the dye increased. Because the DNA detection assays were relatively insensitive, a carrier such as a gel were included to increase the local concentration of the DNA and hence the color signal. Alternatively or additionally, some additional reagent might be added with the dye that would change the color of the post-amplification mix so that the solution containing amplified nucleic acid could be differentiated from one that did not. Dyes that were described by Miyamoto, et al. in US 2011/0117549 were triphenylmethane dye, thiazine dye, oxazine dye, azine dye, xanthene dye, and phenanthridinium dye exemplified by Crystal Violet, Gentian Violet B, Victoria Blue B, Methyl Violet, Night Blue, Methyl Green, Toluidine Blue O, Azure B, Methylene Blue, Brilliant Cresol Blue, Methyl Orange, Pyronin Y. Ethidium Bromide, and Neutral Red.

Subsequent to 2012, others reported pH dependent colorimetric detection of nucleic acids using a wider range of pH dyes and buffers (see US 10,253,357) repeating and extending the earlier work described in US 9,034,606 and US 9,580,148. Although visually detectable dyes were mentioned, these references focused on measuring hue where detecting hue relied on the read-out of a spectrophotometer and various statistical manipulations of the data. This reference recited phenol red, bromocresol purple, bromothymol blue, neutral red, naphtholphthalein, cresol red, cresolphthalein, phenolphthalein, methyl red, and thymolphthalein as examples of pH sensitive dyes. Dye mixtures of phenol red and bromothymol blue, and cresol red and bromothymol blue were also described with buffers having buffering capacities within various pH ranges such as pH 6 - pH 9 or pH 6.5 - pH 8.8.

Although the pH dependent colorimetric amplification assay with phenol red is very effective, it would be desirable to engineer additional improvements. A limitation of the pH dependent colorimetric amplification assay is the limitation on buffer concentration and the required pH range to differentiate positive from negative samples. An unwanted change in the pH might result from the use of dry ice in shipping containers. Certain biofluid compositions such as a subset of saliva samples or certain types of environmental samples might have a less than ideal pH where avoidance or correction of the undesirable pH is limited by the acceptable amount of buffer (e.g., less than 5 mM Tris buffer for phenol red).

Consequently, an alternative dye or dye family that is compatible with polymerases, does not interfere with amplification reactions and provides a strong binary colorimetric signal that translates into a distinct peak for a positive endpoint that is at a different wavelength from a peak at a negative endpoint in the spectrum and is detectable by eye with use in a variety of different amplification reactions not limited to LAMP would be a useful addition to diagnostic test options.

### SUMMARY

Provided herein is a composition comprising an enzyme that releases pyrophosphate from a substrate and a dye of Formula 1: 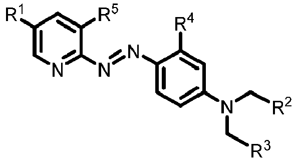 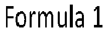 wherein:
R1 and R5 are each independently selected from the group consisting of H, halogen, nitro, cyano, sulfonic acid, carboxyl, trifluoromethyl, trichloromethyl and tribromomethyl;
R2 and R3 are each independently selected from a lower alkyl optionally comprising a terminal sulfonate group; and
R4 is H, hydroxyl or carboxyl.

In some embodiments, at least one of R1 and R5 is a halogen selected from F, CI, Br or I (e.g., Br); R2 and R3 are each independently selected from n-butyl, sec-butyl, isobutyl, tert-butyl, propyl, n-propyl, isopropyl, ethyl, or methyl; and at least one of R2 and R3 comprises the terminal sulfonate group. In some embodiments, R4 is hydroxyl or carboxyl; preferably hydroxyl. In other embodiments, R4 is H.

Embodiments of Formula 1 include: 2-(5-Bromo-2-pyridylazo)-5-(N-propyl-N-sulfopropylamino)phenol (5-Br-PAPS), 2-(5-Nitro-2-pyridylazo)-5-(N-propyl-N-sulfopropylamino)phenol (5-Nitro-PAPS), or 2-(3,5-dibromo-2-pyridylazo)-5-(N-ethyl-N-sulfopropylamino)benzene (3,5-Dibromo-PAESA).

A method for detecting pyrophosphate is also provided. This method may comprise combining: an enzyme (e.g., a polymerase such as a DNA polymerase or an RNA polymerase; or another enzyme that is associated with hydrolysis or hydrolyzes a substrate such as an nucleoside triphosphate (NTP) to release pyrophosphate, such as a reverse transcriptase, a primase, a ligase, a helicase, a nucleotide pyrophosphatase (Ppase)/phosphodiesterase, an RNA decapping enzyme (RNA 5' pyrophosphohydrolase) and a geranyl pyrophosphate synthase); a substrate for the enzyme that releases pyrophosphate when it is hydrolyzed (e.g., NTP); a metal salt (preferably a manganese salt); and a pyridylazoaniline dye (preferably a dye of Formula 1), to produce a reaction mix; incubating the reaction mix under conditions by which the enzyme cleaves the substrate to produce pyrophosphate; and observing a change in color of the reaction mix, wherein the change in color indicates that pyrophosphate has been produced.

In some embodiments, the enzyme is a polymerase and the substrate is an NTP (dNTP or rNTP). For example, the reaction mix may comprise a polymerase, rNTPs or dNTPs, the dye, the metal salt (e.g., manganese salt), and a template nucleic acid; the reaction mix is incubated under conditions suitable for amplification of the nucleic acid, and the change in color indicates that a product has been amplified. Amplification methods may include any of loop-mediated isothermal amplification (LAMP), polymerase chain reaction (PCR), strand displacement amplification (SDA), helicase dependent amplification (HDA), multiple displacement amplification (MDA), rolling circle amplification, ligation mediated amplification, whole genome amplification (WGA), nucleic acid sequence-based amplification (NASBA) or recombinase polymerase amplification (RPA) mix.

A kit comprising an enzyme that releases pyrophosphate from a substrate (e.g., a polymerase), a metal salt (e.g., manganese salt), and a pyridylazoaniline dye (preferably a dye of Formula 1) are also provided.

The present composition, method and kits are believed to be a significant advancement in the art because they provide a way to measure the progress of reactions that release pyrophosphate (e.g., nucleic acid amplification reactions and other reactions that hydrolyze NTPs such as ATP) via a color change. The pyridylazoaniline dyes used in the present disclosure are one color (e.g., red) when they are bound to a divalent metal cation (e.g., Mn²⁺) and another color (e.g., yellow) when they are not bound to the divalent metal cation. Without wishing to be held to any particular theory, it is believed that during these reactions, the liberated pyrophosphate competes with the dye for binding to the metal ion. This competition results in the release of a metal ion from the dye, which is sequestered by the pyrophosphate. Release of the metal ion from the dye restores the dye's original color. In one embodiment, the reaction mix changes color from red to yellow during the course of the reaction, as the pyrophosphate is being produced. The color change can be readily detected by spectrophotometry, image analysis or by eye. By selecting the appropriate detection method, hue or change in peak wavelength can be measured.

### BRIEF DESCRIPTION OF THE FIGURES

The skilled artisan will understand that the drawings, described below, are for illustration purposes only. The drawings are not intended to limit the scope of the present teachings in any way.

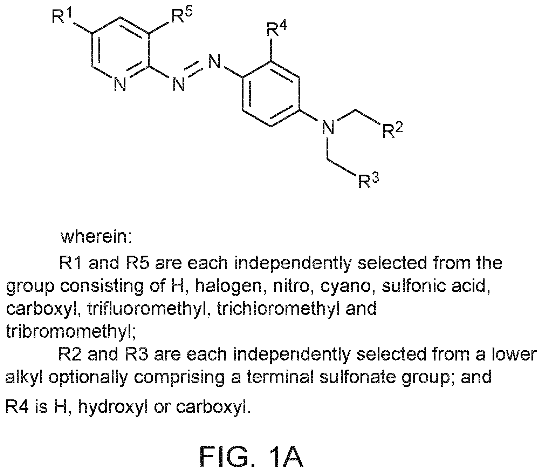

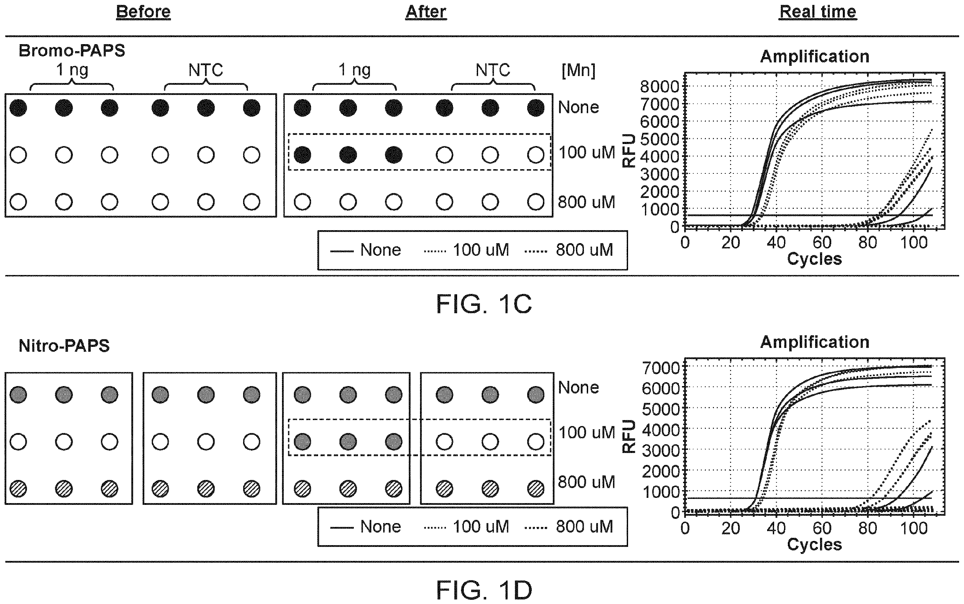

FIGs. 1A-1B shows the structures of a family of dyes identified as 4-(2-pyridylazo)aniline dyes (also referred to herein as pyridylazoaniline dyes) and represented by Formula 1 (FIG. 1A). Examples of this family of dyes show that these dyes perform better in colorimetric LAMP than other azo dyes that are not 4-(2-pyridylazo)aniline dyes (FIGs. 1C-1G).
FIG. 1A shows the structure of a 4-(2-pyridylazo)aniline dye, where:
   R1 and R5 are each independently selected from the group consisting of H, halogen, nitro, cyano, sulfonic acid, carboxyl, trifluoromethyl, trichloromethyl and tribromomethyl; and
   R2 and R3 are each independently selected from a lower alkyl optionally comprising a terminal sulfonate group; and
   R4 is H, OH or COOH.
      In some embodiments, at least one of R1 and R5 is a halogen selected from F, CI, Br or I; and R2 and R3 are each independently selected from n-butyl, sec-butyl, isobutyl, tert-butyl, propyl, n-propyl, isopropyl, ethyl, or methyl; and at least one of R2 and R3 comprises the terminal sulfonate group.

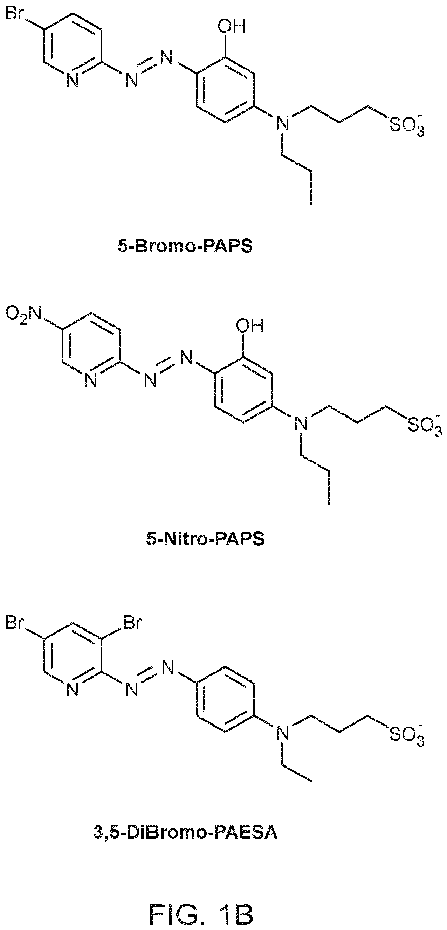

FIG. 1B shows 5-Bromo-PAPs, 5-Nitro-PAPS, and 3,5-DiBromo-PAESA.
FIGs. 1C-1G show two 4-(2-pyridylazo)aniline metal sensing dyes, 5-Bromo-PAPS and 5-Nitro-PAPS ( 2-(2-pyridylazo)-5-(N-propyl-N-sulfopropylamino) phenol that performed significantly better than other non-aniline azo metal sensing dyes 4-(2- pyridylazo)resorcinol) (PAR), hydroxynaphthol blue (HNB) and eriochrome black T (EBT) as indicators of a positive LAMP reaction under the same conditions.
   For each dye, the left plate of 18 wells (before LAMP) and right plate of 18 wells (after LAMP) show the color (in grey-scale) of triplicate LAMP reactions either with lambda DNA (1 ng) or without (NTC). Each dye was tested using 0 µM Mn²⁺ (none), 100 µM Mn²⁺ and 800 µM Mn²⁺. The corresponding real-time curves on the right side of the figure confirm that LAMP occurred. The best visual detection condition for each dye after LAMP is highlighted in the right plate with a dotted rectangle around 6 samples.
FIG. 1C shows the results with 75 µM 5-Bromo- PAPS.
FIG. 1D shows the results with 75 µM 5-Nitro-PAPS.
FIG. 1E shows the results with 200 µM PAR.
FIG. 1F shows the results with 80 µM HNB.
FIG. 1G shows the results with 100 µM EBT.
FIG. 2A-2D show that of the 9 different metal salts tested, manganese ions gave the clearest color change for 5-Bromo-PAPS and 5-Nitro-PAPS.

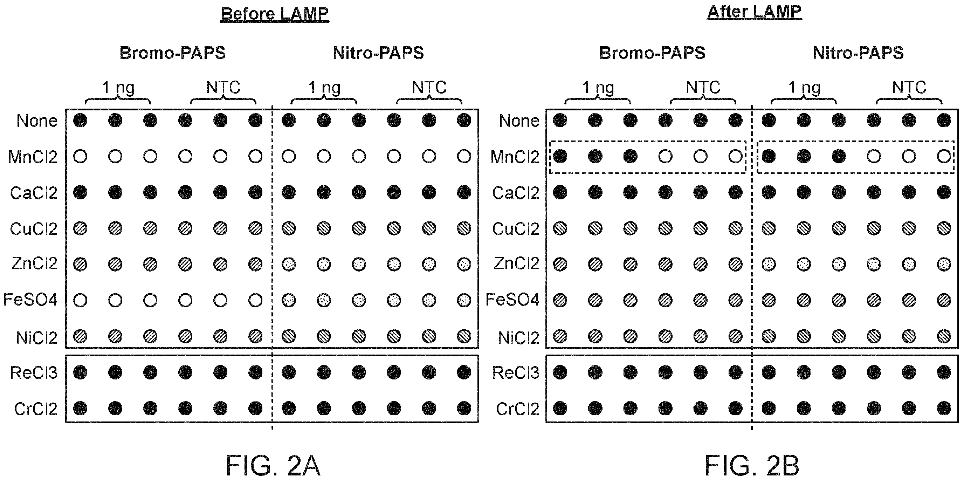

   In FIG. 2A (before LAMP) and FIG. 2B (after LAMP), Mn²⁺ gave an optimal signal with PAPS dyes in response to LAMP amplification using 100 µM metal ion with 75 µM 5- Bromo-PAPS or 5-Nitro-PAPS in triplicates with or without 1 ng Lambda DNA (1 ng). The reactions with Mn²⁺ ions (MnCl₂) are highlighted with dashed rectangles.

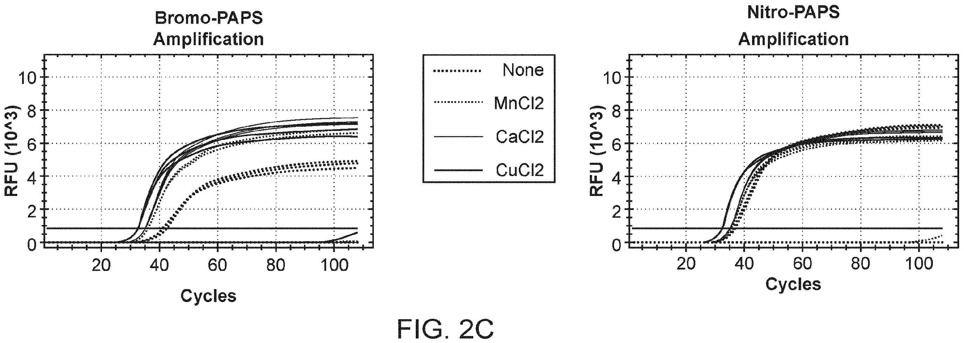

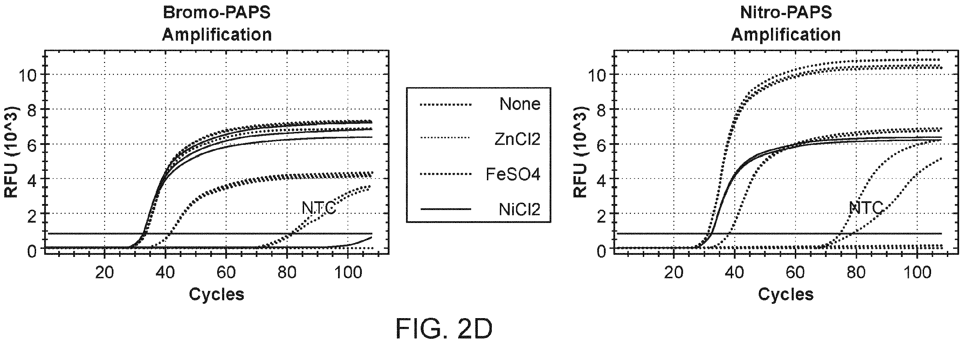

FIG. 2C and FIG. 2D show corresponding real time curves for samples in FIG. 2A and FIG. 2B.

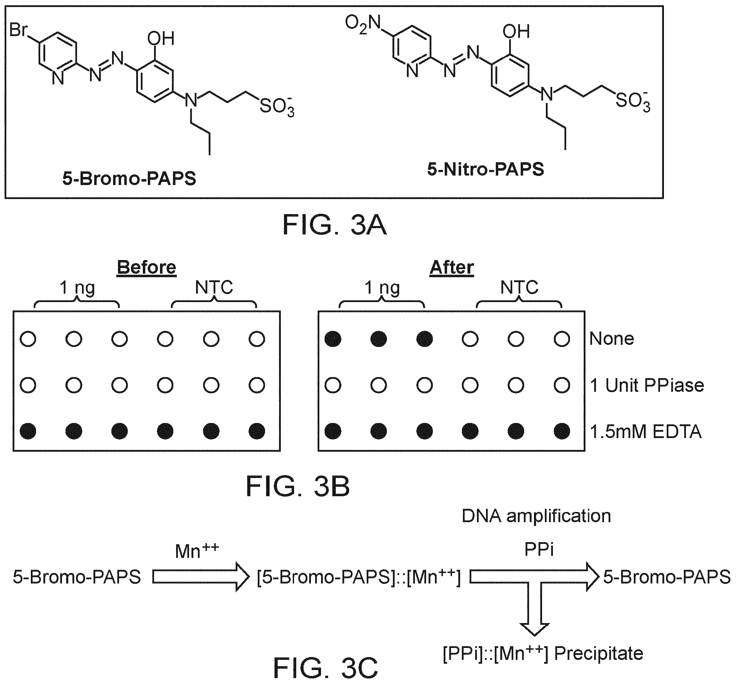

FIGs. 3A-3C show that pyrophosphates released during amplification of DNA trigger a color change of the PAPS dyes by sequestration of Mn²⁺ from a dye:Mn complex. This is confirmed by demonstrating that addition of pyrophosphatase (Ppase) can prevent the color change. Additionally, EDTA mimics an amplification reaction by binding manganese with greater affinity than PAPS in the absence of an amplification reaction resulting in a color change.
FIG. 3A shows the chemical structures of 5-Bromo-PAPS and 5-Nitro-PAPS.
FIG. 3B shows the effects of degrading pyrophosphate by PPase or chelation of Mn²⁺ by EDTA.
FIG. 3C shows the proposed mechanism of color transitions by PAPS dyes during LAMP amplification.

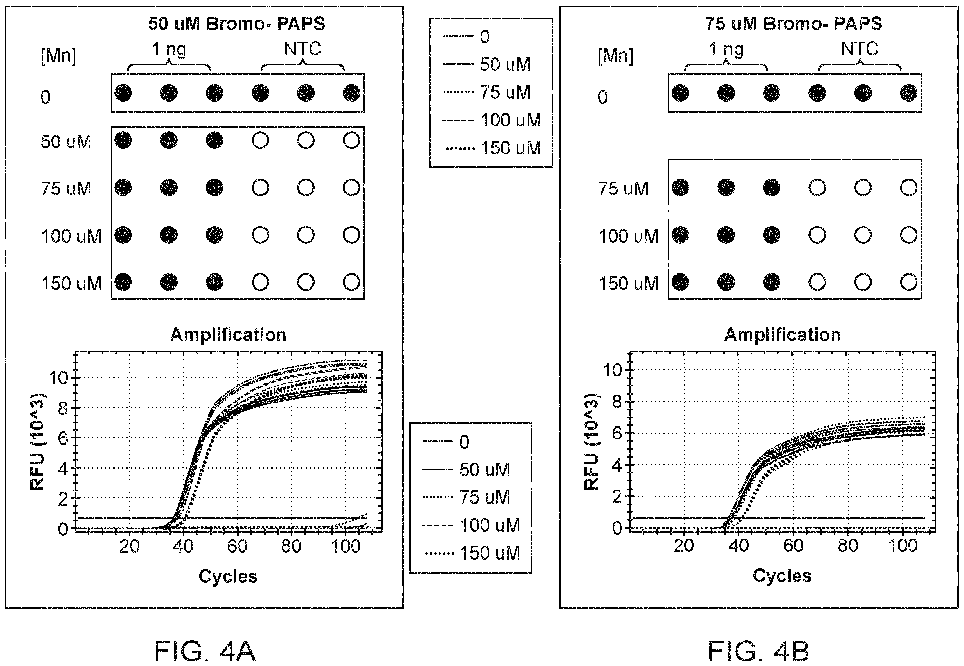

FIGs. 4A-4C show that 50 µM, 75 µM and 100 µM 5-Bromo PAPS provide similar results when tested against 0 µM, 50 µM, 75 µM, 100 µM or 150 µM manganese ions in the LAMP amplification reaction mix.
   The color changes of post-LAMP reactions and the real-time curves for each of the three concentrations of the PAPS dye are provided.
FIG. 4A shows results with 50 µM 5-Bromo-PAPS.
FIG. 4B shows results with 75 µM 5-Bromo-PAPS.

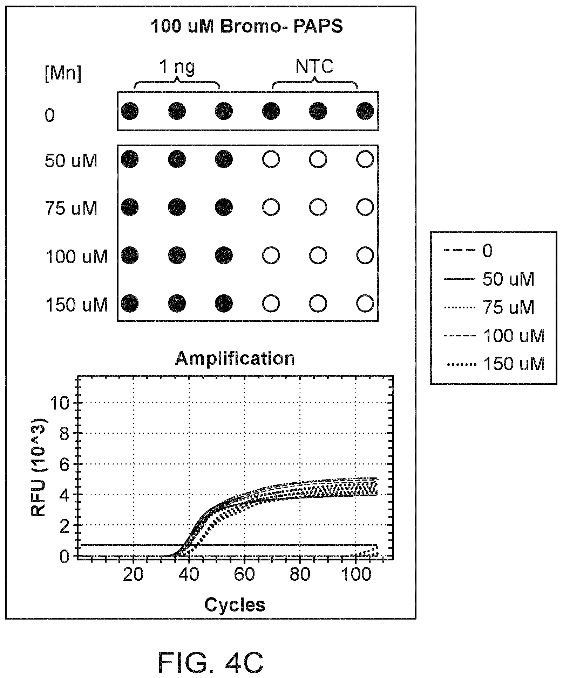

FIG. 4C shows results with 100 µM 5-Bromo-PAPS.

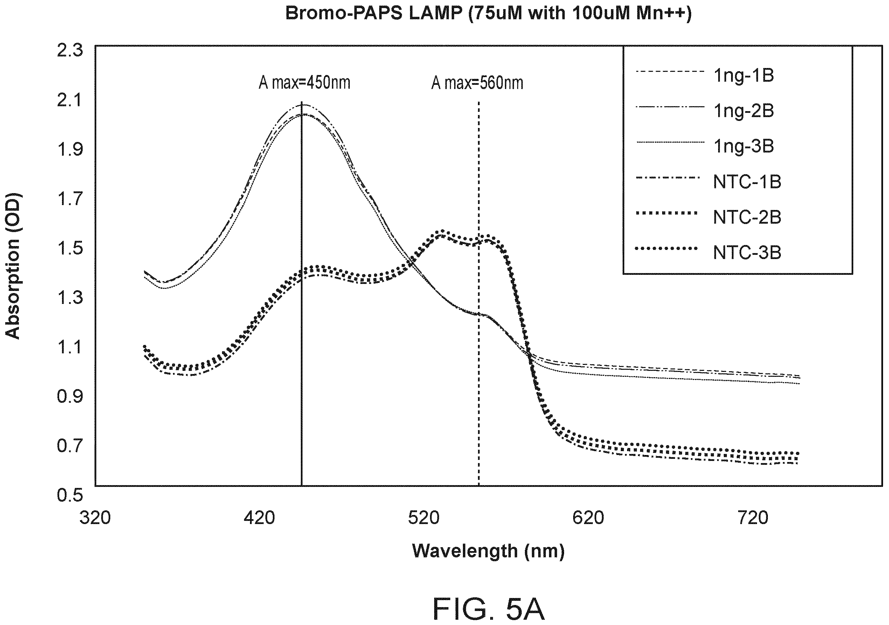

FIGs. 5A-5D show that a color change in the PAPS dye can readily be detected by the absorbance spectrum at peak maxima observed at 450 nm and 570 nm. As can be seen from the curves, a range of spectrums may be used on either side of the peak maxima to detect positive results. For example, wave lengths of 420 nm -500 nm instead of 450 nm and 520 nm-600 nm for 550 nm.
FIG. 5A shows the absorbance measured from 360 nm to 750 nm wavelength in reactions with 75 µM 5-Bromo-PAPS and 100 µM Mn²⁺.

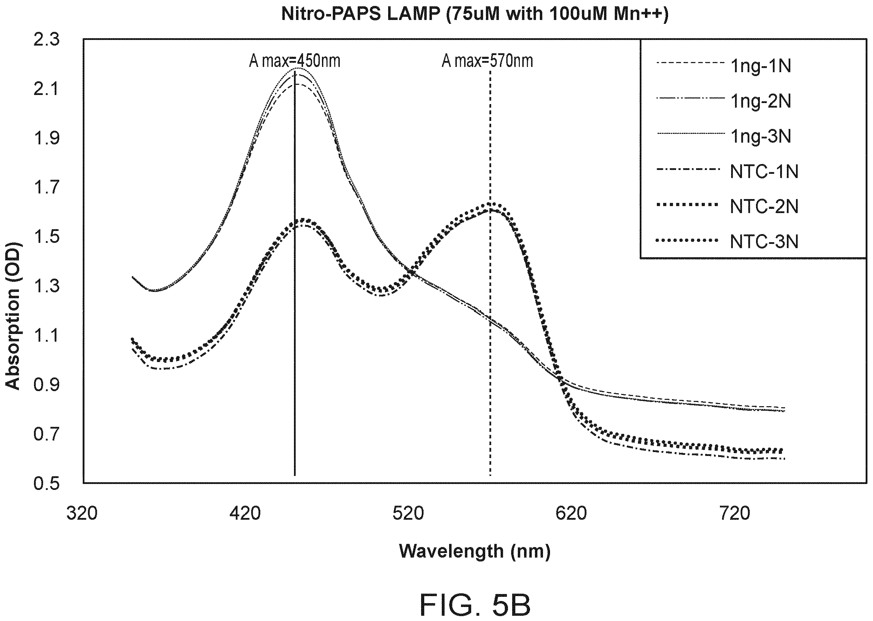

FIG. 5B shows the absorbance measured from 360 nm to 750 nm wavelength in reactions with 75 µM 5-Nitro-PAPS and 100 µM Mn²⁺.

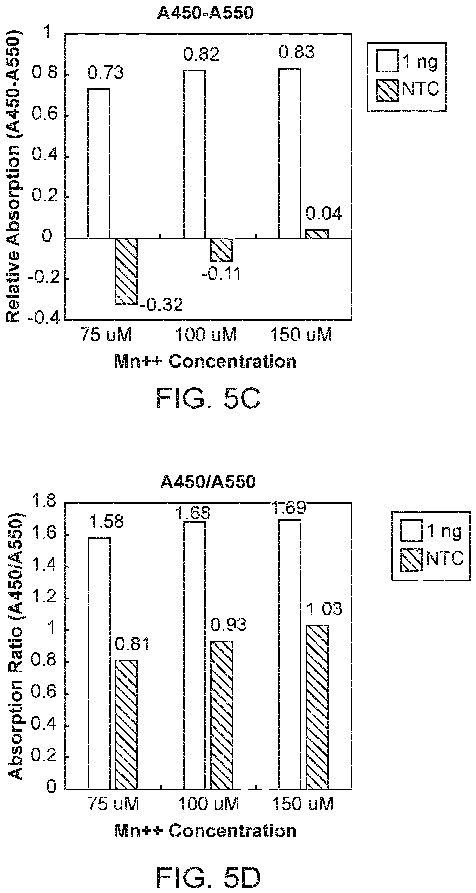

FIG. 5C shows the relative gain of absorbance at 450 nm and 550 nm in reactions with 75 µM 5-Bromo-PAPS and 75 µM -150 µM Mn²⁺.
FIG. 5D shows the ratio of absorbance at 450 nm over 550 nm with 75 µM 5-Nitro-PAPS and 75-150 µM Mn²⁺.

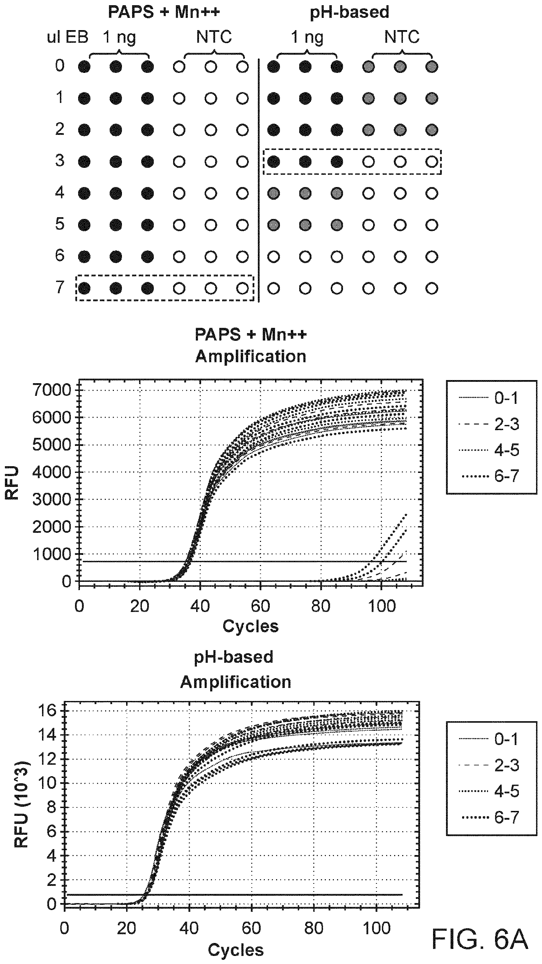

FIGs. 6A-6B show that in a comparison with pH based colorimetric LAMP, the PAPS dye LAMP tolerates more carryover solutions than pH based colorimetric LAMP. The carryover solutions shown here are from standard DNA purification column elution buffer (EB) or virus transport medium (VTM). Similar results are anticipated from carryover of body fluids such as saliva and nasal mucosa.
FIG. 6A shows that where samples 1-7 contain increasing volumes of a carryover buffer, the dotted rectangles show the difference in maximum tolerance to the amount of carryover of EB using the different color assays (PAPs dye using Mn²⁺ as compared to pH-based). Real-time curves (showing different amounts of carryover solution) for LAMP reactions are provided beneath the colored wells (0-7 refers to 0-7 µL (0-28% v/v) of a typical EB (Qiagen EB, 10 mM Tris pH 8.5) in 25 µL LAMP reactions containing the PAPS reporter system).

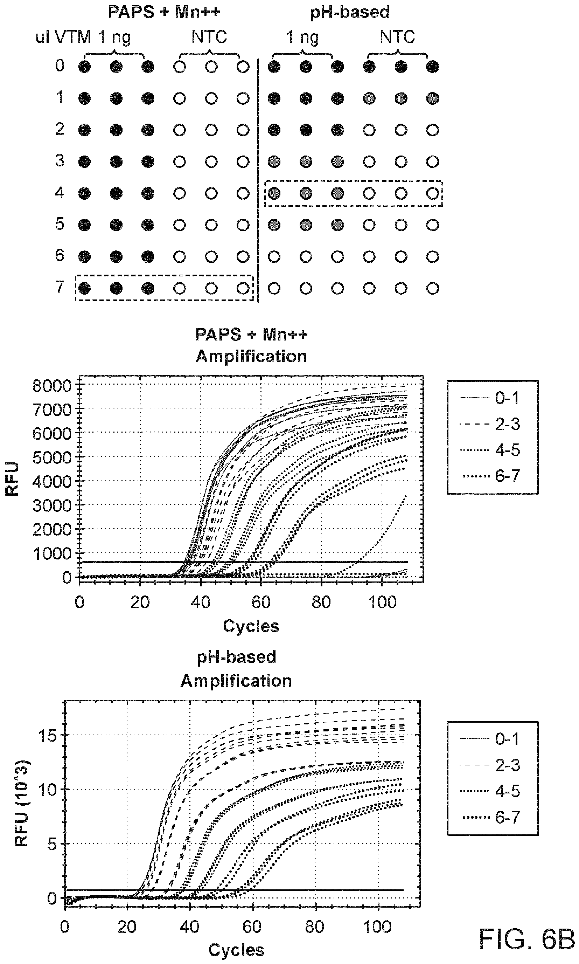

FIG. 6B shows that where samples 1-7 contain increasing volumes of a carryover buffer, the dotted rectangles show the difference in maximum tolerance to the amount of carryover of VTM using the different color assays. Real-time curves (showing different amounts of carryover solution) for LAMP reactions are provided beneath the colored wells.

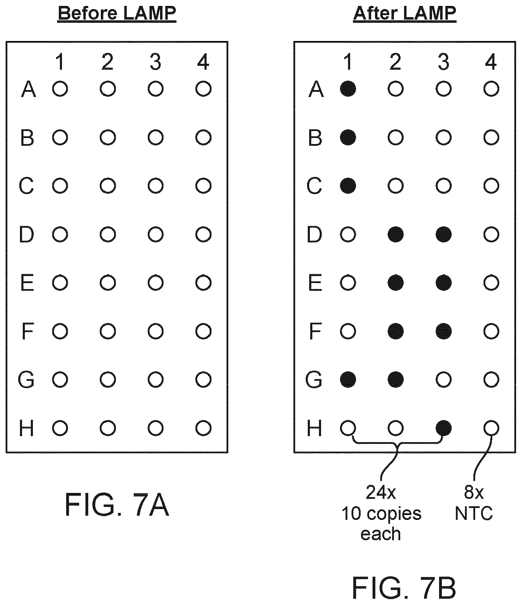

FIGs. 7A-7D show that PAPS dyes are sensitive reporters of amplification using reverse transcription-LAMP (RT-LAMP), E1 primer set and as little as 10 copies of SARS-CoV-2 RNA.
   Results for 24 reactions, each with approximately 10 copies of target RNA (A1-H3) and 8 NTC reactions (A4-H4) in the presence of 75 µM 5-Bromo- PAPS and 100 µM Mn²⁺.
FIG. 7A shows the color of the reaction mix before amplification (the red color is represented by white circles).
FIG. 7B shows the color of the reaction mix after amplification (black circles represent yellow color). The no-template control (NTC) reactions remain red. A color change (to yellow) is observed in reactions A1, B1, C1, D2, D3, E2, E3, F2, F3, G1, G2, and H3.

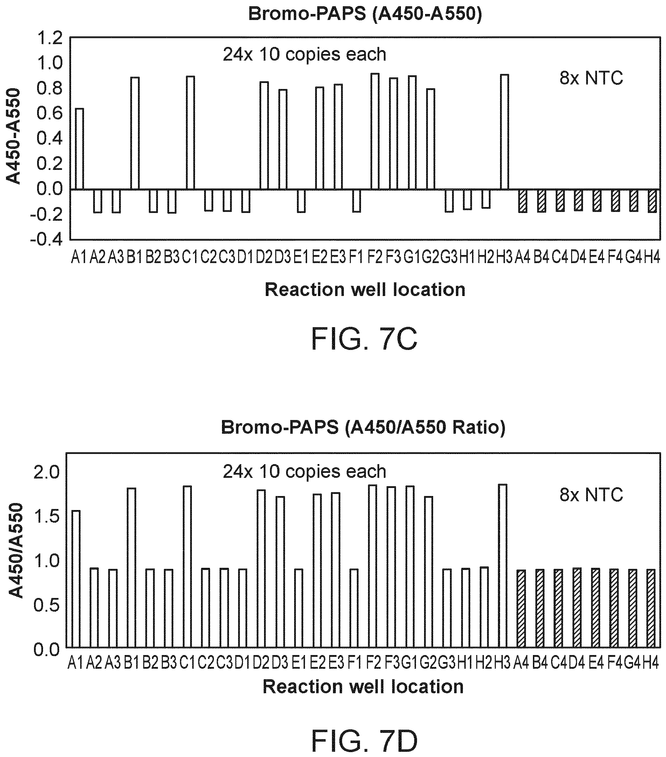

FIG. 7C shows the relative gain of absorbance at 450 nm and 550 nm.
FIG. 7D shows the ratio of absorbance at 450 nm over 550 nm.

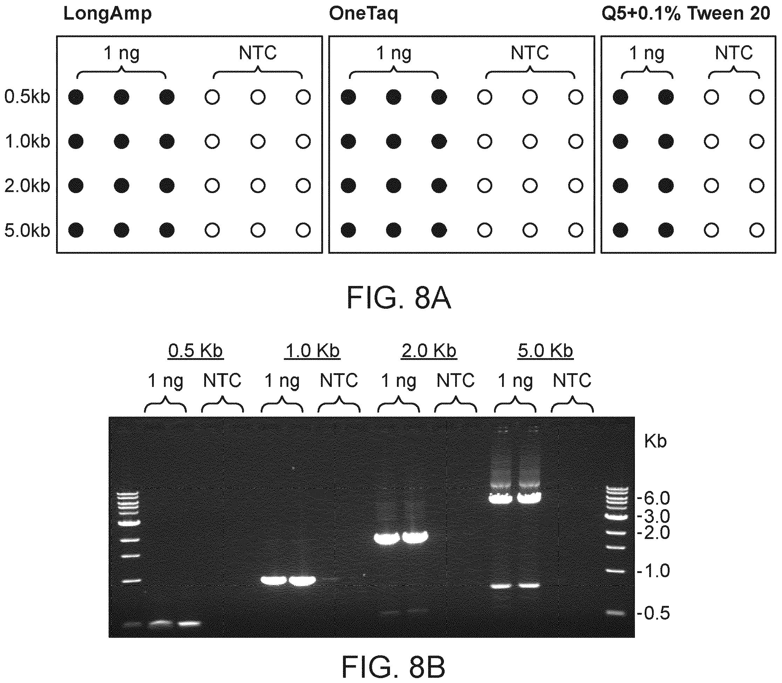

FIGs. 8A-8D shows that PAPS colorimetric detection can be effectively used for PCR of lambda DNA fragments of sizes in the range of 0.5 kb-5.0 kb and is tolerant of various buffers used in different PCR formulations. The results from triplicates samples (LongAmp^{®} and OneTaq^{®} (New England Biolabs, Ipswich, MA)) or duplicate samples (Q5^{®} (New England Biolabs, Ipswich, MA)) in the presence of 50 µM Bromo-PAPS and 50 µM Mn²⁺ are shown.
FIG. 8A shows the different end point dye colors for positive and negative samples (with and without 1 ng DNA template) after PCR with LongAmp Taq, OneTaq, and Q5 master mixes or non-template controls (NTC). All positive samples have a yellow end-point dye color (represented by black circles). All negative samples have a red end-point dye color (represented by yellow circles).
FIG. 8B shows the Q5 PCR products in FIG. 8A on agarose gels after electrophoresis in the presence of the dye where the DNA ranges in size from 0.5 kb, 1.0 kb, 2.0 kb and 5.0 kb.

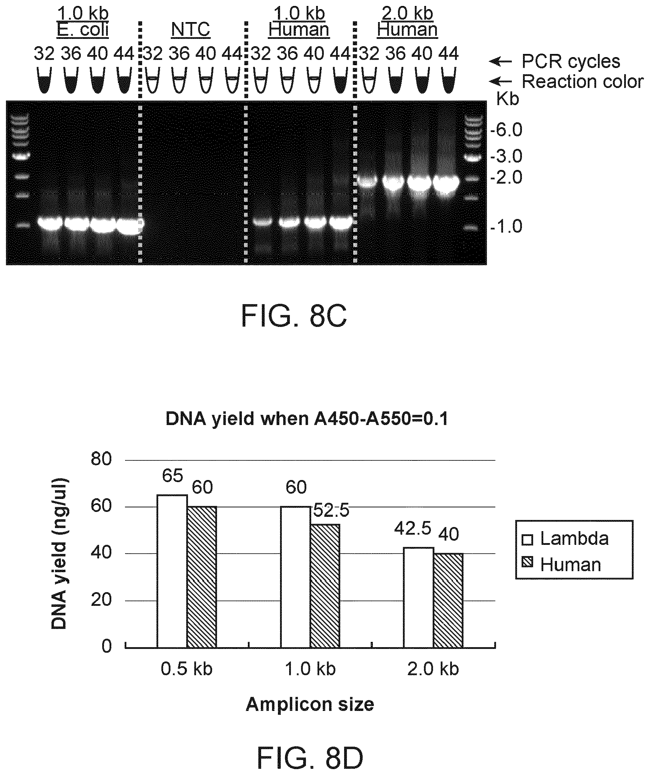

FIG. 8C shows the number of PCR cycles required for a color change from red to yellow when amplifying DNA fragments with OneTaq PCR mix from *E.coli* and human genomes. The reaction color change is shown above the agarose gels containing electrophoresed sample DNA, where yellow color is represented by a black reaction tube, and red color is represented by a white reaction tube.
FIG. 8D shows the DNA yield (ug/µl) determined from A450-A550 ratios for variously sized lambda and human DNA after PCR using OneTaq.

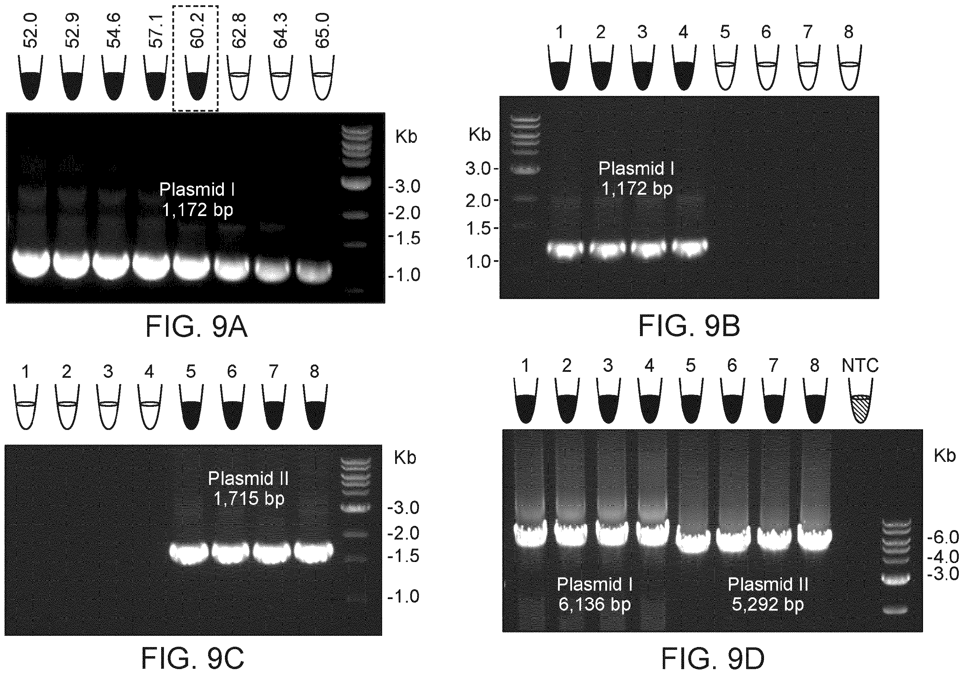

FIGs. 9A-9D show that the colorimetric assay using PAPS dyes may be used directly for colony PCR to assess a target insert in a plasmid or the presence of the plasmid itself. Agarose gels are shown each with the corresponding colorimetric PCR assay above (black tube represents yellow reaction color; white tube represents red reaction color) for *E.coli* colonies where four colonies are tested for plasmid I in FIG. 9A-9B containing a specific insert (1-4), four colonies are tested for plasmid II (1-4) in FIG. 9C and 8 colonies tested for plasmid I and II (FIG. 9D).
FIG. 9A shows optimization of annealing temperature for amplifying an 1172 bp fragment from plasmid I with purified plasmid DNA. The annealing temperature is shown above reaction tubes.
FIG. 9B shows the results of PCR with bacterial cells using specific primers recognizing a DNA insert (1172 bp) in a plasmid (plasmid I) in the presence of a colorimetric dye and comparing this to the same DNA obtained on a gel.
FIG. 9C shows the results of PCR with bacterial cells using specific primers recognizing a different DNA insert (1715 bp) in a plasmid (plasmid II) in the presence of a colorimetric dye and comparing this to the same DNA obtained on a gel.
FIG. 9D shows PCR with bacterial cells using primers recognizing both plasmid I (6136 bp) and II (5292bp).

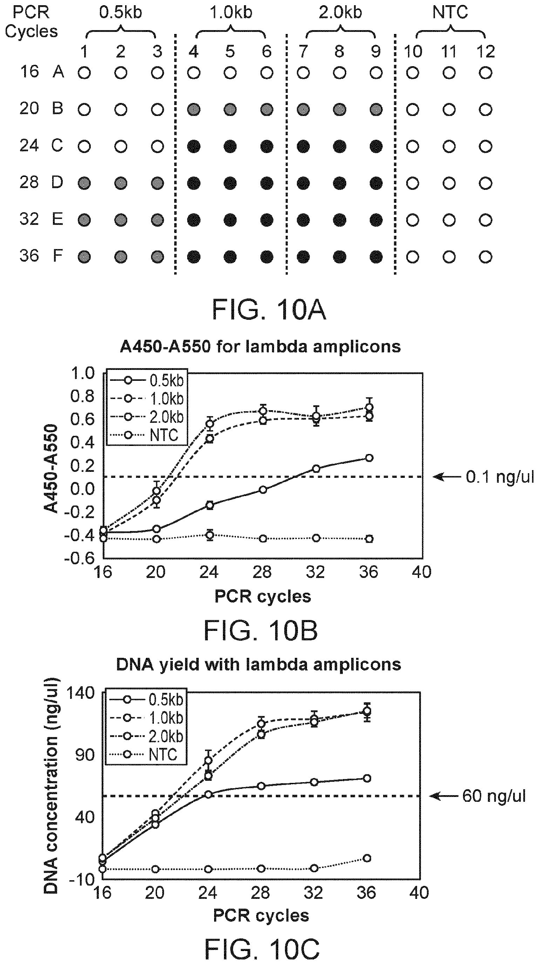

FIGs. 10A-10C show that the concentration of Lambda DNA in a sample can be determined by determining the number of cycles of PCR required to obtain a desired intensity of color change to yellow in the sample for a predetermined size of the amplicon.
FIG. 10A shows a time course of color changes of PAPS dye for triplicate PCR reactions after 16, 20, 24, 28, 32 and 36 PCR cycles using 0.5 kb, 1.0 kb and 2.0 kb Lambda DNA at a starting concentration of 1 ng lambda DNA (~1.9x10⁷ copies) and a NTC control reactions with primers for the 0.5 kb DNA in the absence of the template DNA.
FIG. 10B shows spectrophotometric scans of the samples from FIG. 10A at an absorbance at 450 nm and 550 nm, plotted against the PCR cycle numbers. An arbitrary threshold (dashed line) of 0.10 was drawn in the graph for visible color change perceivable by the naked eye.
FIG. 10C shows DNA yield in the reactions from FIG. 10A. The visual color change threshold (dashed line) corresponds to ~ 60 ng/µl amplicon yield in the PCR reactions.

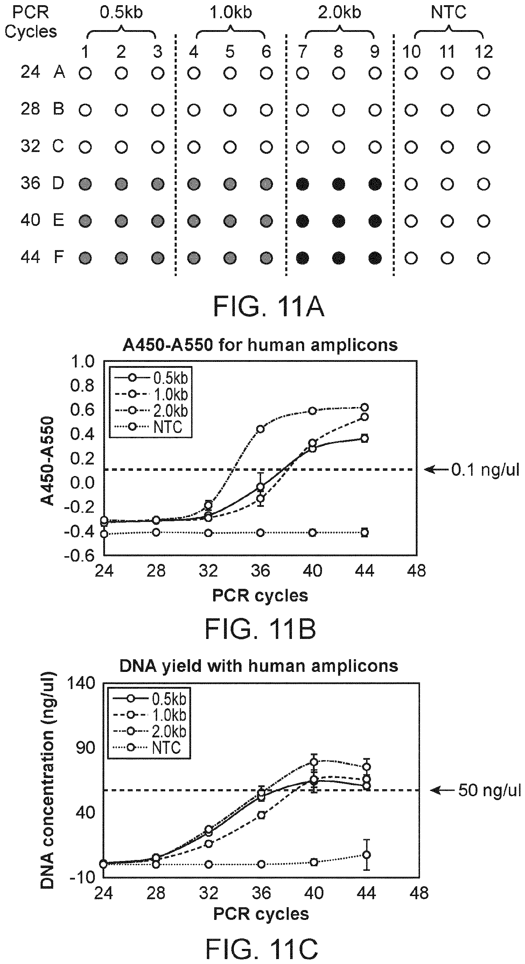

FIGs. 11A-11C show that the concentration yield of human DNA in a sample can be determined by PAPS dye color and correlated to the time course of PCR required to obtain a desired intensity of color change to yellow in the sample.
FIG. 11A shows a time course of color changes of PAPS dye for triplicate PCR reactions after 24, 28, 32, 36, 40 and 44 cycles using 0.5 k, 1.0 kb and 2.0 kb Human DNA at a starting concentration of 10 ng human DNA (^{~}2,900 copies) and a NTC control reactions with primers for the 0.5 kb DNA in the absence of the template DNA. More PCR cycles were used with human versus lambda DNA because of the reduced template copy numbers.
FIG. 11B shows spectrophotometric scans of the samples from FIG. 11A at an absorbance at 450 nm and 550 nm, plotted against the PCR cycle numbers. An arbitrary threshold (dashed line) of 0.10 was drawn in the graph for visible color change perceivable by the naked eye.
FIG. 11C shows DNA yield in the reactions from FIG. 11A. The visual color change threshold (dashed line) corresponds to ^{~} 50 ng/µl amplicon yield in the PCR reactions.

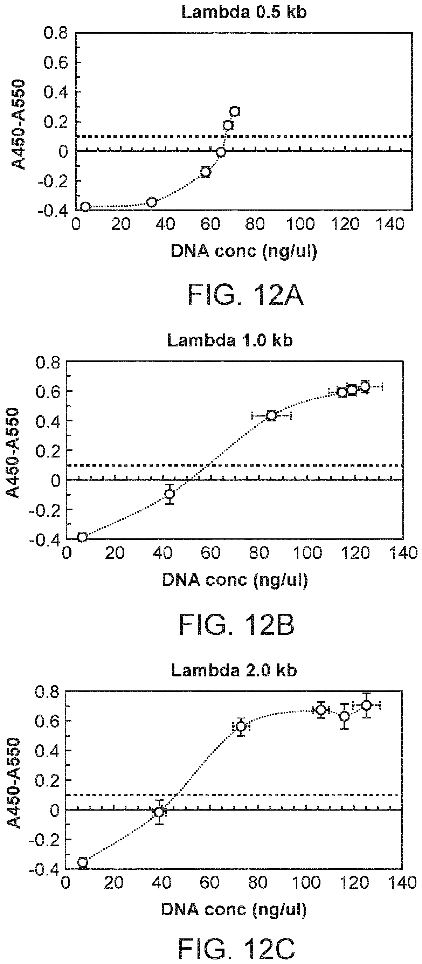

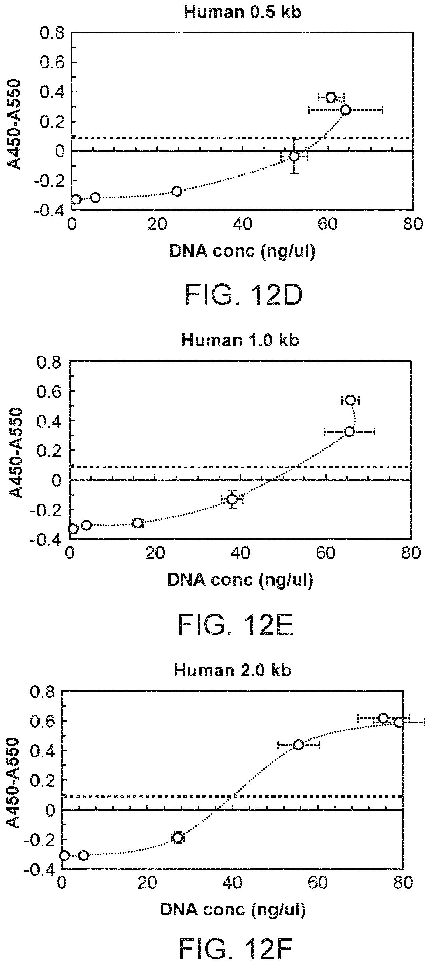

FIGs. 12A-12F shows a useful correlation of color change with DNA yield in PCR reactions. The average absorbance gains (A450-A550) (Y-axis) of PCR reactions were plotted against DNA yield (X-axis) for lambda (A, B, C) and human (D, E, F) amplicons along 5 sampling points of PCR reactions (16, 20, 24, 28, 32 and 36 cycles for lambda amplicons and 24, 28, 32, 36, 40 and 44 cycles for human amplicons). The horizontal red dashed line corresponding to A450-A550 of 0.10, which is a point where a color change is perceivable by the naked eye. The DNA concentrations intercepted with this threshold were obtained as the DNA yield required to see color change and depicted in FIG. 8D.

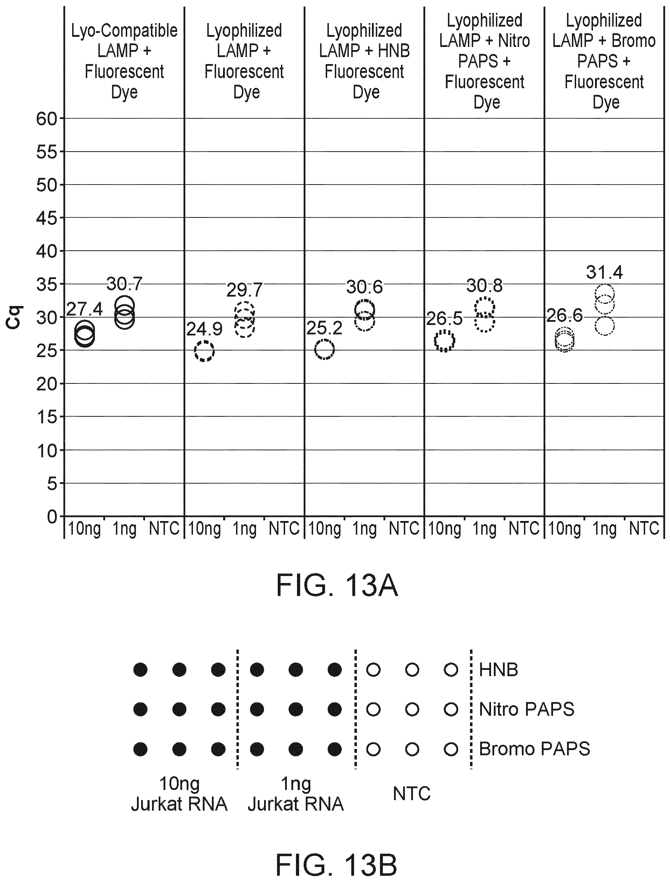

FIGs. 13A-13B show that the lyophilized PAPS dye RT-LAMP reaction provides similar results to lyophilized fluorescent dye LAMP and Lyo compatible fluorescent dye LAMP that has not been lyophilized, under the same reaction conditions using Jurkat RNA. However, when HNB dye is used in a lyophilized mixture, it fails to reveal a color change after the amplification reaction is completed.

### DETAILED DESCRIPTION

Unless otherwise defined, all technical and scientific terms used herein have the same meaning as commonly understood by one of ordinary skill in the art to which this disclosure belongs. Still, certain terms are defined herein with respect to embodiments of the disclosure and for the sake of clarity and ease of reference.

Sources of commonly understood terms and symbols may include: standard treatises and texts such as Kornberg and Baker, DNA Replication, Second Edition (W.H. Freeman, New York, 1992); Lehninger, Biochemistry, Second Edition (Worth Publishers, New York, 1975); Strachan and Read, Human Molecular Genetics, Second Edition (Wiley-Liss, New York, 1999); Eckstein, editor, Oligonucleotides and Analogs: A Practical Approach (Oxford University Press, New York, 1991); Gait, editor, Oligonucleotide Synthesis: A Practical Approach (IRL Press, Oxford, 1984); Singleton, et al., Dictionary of Microbiology and Molecular biology, 2d ed., John Wiley and Sons, New York (1994), and Hale & Markham, the Harper Collins Dictionary of Biology, Harper Perennial, N.Y. (1991) and the like.

As used herein and in the appended claims, the singular forms "a," "an," and "the" include plural referents unless the context clearly dictates otherwise. For example, the term "a protein" refers to one or more proteins, i.e., a single protein and multiple proteins. The claims can be drafted to exclude any optional element when exclusive terminology is used such as "solely," "only" are used in connection with the recitation of claim elements or when a negative limitation is specified.

Aspects of the present disclosure can be further understood in light of the embodiments, section headings, figures, descriptions and examples, none of which should be construed as limiting the entire scope of the present disclosure in any way. Accordingly, the claims set forth below should be construed in view of the full breadth and spirit of the disclosure.

Each of the individual embodiments described and illustrated herein has discrete components and features which may be readily separated from or combined with the features of any of the other several embodiments without departing from the scope or spirit of the present teachings. Any recited method can be carried out in the order of events recited or in any other order which is logically possible.

Numeric ranges are inclusive of the numbers defining the range. All numbers should be understood to encompass the midpoint of the integer above and below the integer i.e. the number 2 encompasses 1.5-2.5. The number 2.5 encompasses 2.45-2.55 etc. When sample numerical values are provided, each alone may represent an intermediate value in a range of values and together may represent the extremes of a range unless specified.

In the context of the present disclosure, "non-naturally occurring" refers to a polynucleotide, polypeptide, carbohydrate, lipid, or composition that does not exist in nature. Such a polynucleotide, polypeptide, carbohydrate, lipid, or composition may differ from naturally occurring polynucleotides polypeptides, carbohydrates, lipids, or compositions in one or more respects. For example, a polymer (e.g., a polynucleotide, polypeptide, or carbohydrate) may differ in the kind and arrangement of the component building blocks (e.g., nucleotide sequence, amino acid sequence, or sugar molecules). A polymer may differ from a naturally occurring polymer with respect to the molecule(s) to which it is linked. For example, a "non-naturally occurring" protein may differ from naturally occurring proteins in its secondary, tertiary, or quaternary structure, by having a chemical bond (e.g., a covalent bond including a peptide bond, a phosphate bond, a disulfide bond, an ester bond, and ether bond, and others) to a polypeptide (e.g., a fusion protein), a lipid, a carbohydrate, or any other molecule. Similarly, a "non-naturally occurring" polynucleotide or nucleic acid may contain one or more other modifications (e.g., an added label or other moiety) to the 5'- end, the 3' end, and/or between the 5'- and 3'-ends (e.g., methylation) of the nucleic acid. A "non-naturally occurring" composition may differ from naturally occurring compositions in one or more of the following respects: (a) having components that are not combined in nature, (b) having components in concentrations not found in nature, (c) omitting one or components otherwise found in naturally occurring compositions, (d) having a form not found in nature, e.g., dried, freeze dried, crystalline, aqueous, and (e) having one or more additional components beyond those found in nature (e.g., buffering agents, a detergent, a dye, a solvent or a preservative).

### Dyes according to Formula 1

The dye used in all aspects of the invention has a structure of Formula 1 (also named 4-(2-pyridylazo)aniline), having the structure shown below: 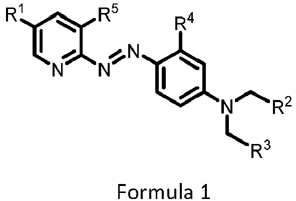 wherein:
R1 and R5 are each independently selected from the group consisting of H, halogen, nitro, cyano, sulfonic acid, carboxyl, trifluoromethyl, trichloromethyl and tribromomethyl;
R2 and R3 are each independently selected from a lower alkyl optionally comprising a terminal sulfonate group; and
R4 is H, hydroxyl or carboxyl.

Dyes of Formula 1 are a family of commercially available 4-(2-pyridylazo)aniline dyes (also referred to herein as pyridylazoaniline dyes).

Preferred compounds according to structural Formula (I), including any associated counterions, are water-soluble azo dyes. Particularly preferred water-soluble azo dyes comprise a sulfonate group. Most preferred azo dyes are pyridylazo dyes which have an electron-withdrawing substituent at R1 position such as halogen, nitro, cyano, sulfonic acid, trifluoromethyl and trichloromethyl, most preferably bromo or nitro.

In some embodiments, at least one of R1 and R5 is nitro. In one embodiment, R1 is nitro.

In some embodiments, at least one of R1 and R5 is a halogen independently selected from F, CI, Br and I. For example, at least one of R1 and R5 may independently be selected from F, Cl and Br. In embodiments, at least one of R1 and R5 is Br (e.g., R1 is Br).

In some embodiments , R1 and R5 are each independently selected from F, CI, Br and I. For example, R1 and R5 may each independently be selected from F, Cl and Br. In one embodiment, R1 and R5 are both Br.

In other embodiments, one of R1 and R5 is a halogen selected from F, CI, Br and I, and the other is selected from H, nitro, sulfonic acid, and carboxyl. For example, one of R1 and R5 may be selected from F, Cl and Br (e.g., Br) and the other may be selected from H and nitro (e.g., H).

In some embodiments , R1 is a halogen selected from F, CI, Br and I. For example, R1 may be selected from F, Cl and Br (e.g., Br). In some embodiments where R1 is a halogen, R5 is independently selected from F, CI, Br and I. For example, where R1 is a halogen R5 may independently be selected from F, Cl and Br (e.g., Br). In other embodiments where R1 is a halogen, R5 is selected from H, nitro, cyano, sulfonic acid, carboxyl, trifluoromethyl, trichloromethyl and tribromomethyl. For example, where R1 is a halogen, R5 may be selected from H, nitro, sulfonic acid and carboxyl (e.g., H or nitro). In one embodiment where R1 is a halogen, R5 may be H. In one embodiment, R1 is Br and R5 is H. In another embodiment, R1 is Br and R5 is Br. In another embodiment, R1 is Br and R5 is nitro.

In some embodiments , R5 is a halogen selected from F, CI, Br and I. For example, R5 may be selected from F, Cl and Br (e.g., Br). In some embodiments where R5 is a halogen, R1 is independently selected from F, CI, Br and I. For example, where R5 is a halogen R1 may independently be selected from F, Cl and Br (e.g., Br). In other embodiments where R5 is a halogen, R1 is selected from H, nitro, cyano, sulfonic acid, carboxyl, trifluoromethyl, trichloromethyl and tribromomethyl. For example, where R5 is a halogen, R1 may be selected from H, nitro, sulfonic acid and carboxyl (e.g., H or nitro). In one embodiment where R5 is a halogen, R1 may be H. In one embodiment, R5 is Br and R1 is H. In another embodiment, R5 is Br and R1 is Br. In another embodiment, R5 is Br and R1 is nitro.

In some embodiments, R2 and R3 are each independently a lower alkyl with 1 to 10 carbon atoms (C₁₋₁₀-alkyl), preferably from 1 to 4 carbon atoms (C₁₋₄-alkyl), which may be linear or branched, and which may optionally have a terminal sulfonate group. For example, in some embodiments the R2 and R3 groups are each independently selected from: butyl, such as n-butyl, sec-butyl, isobutyl, or tert-butyl; propyl, such as n-propyl or isopropyl; ethyl; or methyl; each of which is optionally and independently substituted by a terminal sulfonate group.

In some embodiments, the R2 and R3 groups are each independently n-butyl, sec-butyl, isobutyl, tert-butyl, propyl, n-propyl, isopropyl, ethyl, or methyl, each of which is optionally and independently substituted by a terminal sulfonate group. Preferably, R2 and R3 are each independently selected from ethyl and methyl, each of which is optionally and independently substituted by a terminal sulfonate group. In one embodiment, R2 and R3 are both ethyl, each optionally and independently substituted by a terminal sulfonate group. In another embodiment, one of R2 and R3 is ethyl and the other is methyl, each optionally and independently substituted by a terminal sulfonate group. In a further embodiment, R2 and R3 are both methyl, each optionally and independently substituted by a terminal sulfonate group.

In some embodiments, at least one of R2 and R3 comprises the terminal sulfonate group. In other embodiments, only one of R2 and R3 comprises the terminal sulfonate group. In further embodiments, neither of R2 and R3 comprises the terminal sulfonate group.

Preferably, R2 and R3 are each independently selected from: butyl, such as n-butyl, sec-butyl, isobutyl, or tert-butyl; propyl, such as n-propyl or isopropyl; ethyl; or methyl, wherein one of R2 and R3 is substituted by a terminal sulfonate group. Most preferably, R2 and R3 are each independently selected from methyl and ethyl, wherein one of R2 and R3 (e.g., R2) is substituted by a terminal sulfonate group. In one embodiment, one of R2 and R3 is ethyl and the other is ethyl substituted by a terminal sulfonate group. In another embodiment, one of R2 and R3 is methyl and the other is ethyl substituted by a terminal sulfonate group.

In some embodiments, R4 is hydroxyl or carboxyl. In other embodiments, R4 is H. Preferably, R4 is hydroxyl.

A mono- or bicyclic heteroaromatic group may be selected from 2H-pyrrolyl, pyrrolyl, imidazolyl, benzimidazolyl, pyrazolyl, indazolyl, purinyl, 8-azapurinyl, pyridyl, pyrazinyl, pyrimidinyl, pyridazinyl, 4H-quinolizinyl, isoquinolyl, quinolyl, phthalazinyl, naphthyridinyl, quinoxalyl, quinazolinyl, quinolinyl, pteridinyl, indolizinyl, 3H-indolyl, indolyl, isoindolyl, oxazolyl, isoxazolyl, thiazolyl, isothiazolyl, tetrazolyl, furazanyl, thienyl, furanyl, or benzo[d]pyrazolyl. More preferably the mono- or bicyclic heteroaromatic group is selected from the group consisting of pyridyl, e.g., 2-, 3- or 4-pyridyl, and thiazolyl; e.g., 2-thiazolyl.

An acyl group may be lower alkylcarbonyl with 1 to 5 carbon atoms (C1-C5), e.g., acetyl, propionyl, n- or isopropylcarbonyl, or n-, iso- or tert-butylcarbonyl, or arylcarbonyl, e.g., benzoyl.

Examples of preferred compounds according to structural Formula I are: 2-(5-bromo-2-pyridylazo)-5-[N-propyl-N-(3-sulfopropyl)amino]phenol disodium salt dihydrate (abbreviated as 5-Bromo-PAPS) and 2-(5-nitro-2-pyridylazo)-5-[N-propyl-N-(3-sulfopropyl)amino]phenol disodium salt dihydrate (abbreviated as 5-Nitro-PAPS). The dye of Formula 1 may alternatively be 3,5-Dibromo-PAESA.

Working examples are provided herein for pyridylazoaniline compounds of Formula I (FIG. 1A) more specifically dyes described in FIG. 1B for PCR and for LAMP.

Dyes according to Formula 1 bind metal ions to form dye: metal complexes and have been selected from a wide range of structures to provide an improvement to existing colorimetric detection of molecular biology reactions.

Pyridylazoaniline compounds of Formula I (FIG. 1A), more specifically dyes described in FIG. 1B, have phenolic and pyridylazo moieties with sulfonate groups that enhance water solubility. These dyes may form complexes with various metal ions such as Mn(II), Ca(II), Fe(II), Re(II), Co(III), Cr(III), Cu(II), Ni(II), Mn(II) and Zn(II) through coordination with oxygen of phenolic group and nitrogens of azo and pyridyl groups. In all aspects of the invention, the metal ion is (the metal salt contains) any metal ion that is (i) capable of forming a complex with the dye of Formula 1, and (ii) capable of being removed from the dye-metal complex in the presence of pyrophosphate. The metal ion is preferably a divalent manganese ion (Mn²⁺).

In some embodiments, a dye according to structural Formula I is combined with one or more divalent metal ions selected from the group of Mg(II), Mn(II), Fe(II), Co(II), Ni(II),Cu(II), and Zn(II), preferably Mn(II). The dye may be combined with the selected divalent metal ion such as Mn(II) in the form of a salt (e.g., MnCl₂, MnSO₄, MnCO₃, or Mn(NO₃)₂; preferably MnCl₂). In some embodiments of the invention, a dye according to structural Formula I is complexed with one or more divalent metal ions selected from the group of Mg(II), Mn(II), Fe(II), Co(II), Ni(II),Cu(II), and Zn(II), preferably Mn(II), to form a dye:metal complex, where the divalent metal ion is capable of being removed from the dye:metal complex in the presence of pyrophosphate.

This family of dyes are shown here to change color in the presence of pyrophosphate. Pyrophosphate is a product of many enzyme reactions. For example, pyrophosphate is released from nucleoside triphosphates (NTP) (NTPs, such as dNTPs or rNTPs, including ATP (the energy source)) when NTPs are hydrolysed to nucleoside monophosphates (NMPs), such as during nucleic acid amplification.

Pyrophosphate released from a substrate (e.g., NTP) by the action of an enzyme competes with the pyridylazoaniline dye for binding to the metal ion. The result is the release of the metal ion from the dye and the binding of the metal ion to pyrophosphate to form an insoluble precipitate (e.g., binding of Mn²⁺ to pyrophosphate forms insoluble manganese(II) pyrophosphate). Without wishing to be bound by theory, the pyrophosphate sequesters the metal ion away from dye-metal complexes resulting in a detectable hypsochromic shift. Preferably the dye changes its absorbance characteristics (color) in response to loss of metal ions from the pyridylazoaniline-metal ion complex, so that the dye-metal ion complex shows absorbance characteristics at one wavelength while the dye alone after release of the metal ion has absorbance characteristics at a second wavelength that is distinct and distinguishable from the first wavelength. The dye:metal ion complex therefore has a modified absorption signal as compared to the uncomplexed dye.

Thus, the dye-metal ion complex is one color, and the dye alone is a distinct and distinguishable color from the dye-metal ion complex (e.g., when complexed with Mn²⁺, 5-Bromo-PAPS is red and 5-Nitro-PAPS is purple-brown; whereas both these dyes appear yellow when uncomplexed). The release of the metal ion from the dye and the binding of the metal ion to pyrophosphate restores the dye's original absorption signal and hence color (e.g., a color change to yellow for 5-Bromo-PAPS and 5-Nitro-PAPS).

Consequently, this family of dyes can be used as an indicator that a specific enzyme reaction has occurred. In one embodiment, the dye of Formula 1 is used to determine reverse transcription of a target RNA, or to detect RNA and/or DNA generated by isothermal DNA amplification or PCR DNA amplification methods. It is desirable that color change of the dye that relies on a metal salt does not interfere with the activity of the enzyme to perform the reaction.

The observed improvements achieved with dyes of Formula 1 include an unambiguous difference in visible color between a sample in the presence of pyrophosphate (e.g., yellow) and a sample where no pyrophosphate is formed (e.g., red or purple-brown) that also correspond to significantly different wavelengths for spectroscopic analysis.

In preferred embodiments, divalent manganese ions were selected for improved sensitive and binary color change (see FIG. 2A-2D). 5-Bromo-PAPS and 5-Nitro-PAPS were shown in FIG. 2A-2B to provide the desired visually detected binary color change (from red to yellow) in the presence of Mn²⁺ ions. In FIG. 2A-2B, manganese chloride was tested. However, manganese sulfate, nitrate, chloride, carbonate or any other salt of manganese would be expected to be effective in enabling a dye-manganese complex to form the observed red or purple-brown color and the manganese to be displaced by pyrophosphate to change the dye color to yellow. The color of the dyes of Formula 1, more specifically the dyes in FIG. 1B, without Mn²⁺ or after sequestration of manganese ion is yellow. At the pH of amplification reactions, the metal ion-dye complexes are red and purple-brown for 5-Bromo-PAPS and 5-Nitro-PAPS, respectively.

The dyes of Formula 1 exemplified in FIG. 1B are shown herein to be sensitive to the production of pyrophosphates by hydrolysis of NTPs, such as during amplification of nucleic acids. This sensitivity was identified using any of a plurality of amplification methods. These dyes enabled detection of relatively low concentrations of nucleic acid substrate and thus proved suitable for diagnostic detection of nucleic acids that are present at low concentrations in the environment. The dyes of Formula 1 exemplified in FIG. 1B also enabled quantification of target nucleic acids and amplicon yield in purified or crude samples. These dyes were not adversely affected by pH changes nor by the presence of a variety of contaminants such as carry over buffers from columns, viral transport medium (VTM), body fluids such as saliva or nasal mucosa containing the nucleic acid, cell lysates, or from in vitro translation mixes. Other advantages include the ability of the dyes to be lyophilized with or without other reagents.

### Enzymes that release pyrophosphate from a substrate

In all aspects of the invention, the enzyme that releases pyrophosphate from a substrate may be any enzyme that cleaves a substrate, wherein cleavage of the substrate by the enzyme results in the production of pyrophosphate. For example, the enzyme may hydrolyze a nucleoside triphosphate (NTP) substrate, which may be a dNTP or an rNTP (e.g., ATP), wherein hydrolysis of the NTP releases pyrophosphate.

Examples of enzymes that release pyrophosphate by hydrolysis of an NTP include a DNA polymerase, an RNA polymerase, a reverse transcriptase, a primase, a ligase, a helicase, a nucleotide pyrophosphatase (Ppase)/phosphodiesterase, an RNA decapping enzyme (RNA 5' pyrophosphohydrolase) or a geranyl pyrophosphate synthase. The enzyme is preferably a DNA polymerase, which may be a thermostable DNA polymerase or a strand displacing DNA polymerase; an RNA polymerase; or a reverse transcriptase. In some embodiments, the enzyme is a DNA polymerase. For example, the DNA polymerase may be a Bst polymerase, Bsu polymerase, archaeal DNA polymerase, Taq polymerase, or a variant thereof.

### Compositions

There is provided a composition comprising an enzyme that releases pyrophosphate from a substrate and a pyridylazoaniline dye; preferably a dye of Formula 1. The enzyme may be any enzyme as defined above, preferably a DNA polymerase, RNA polymerase, or reverse transcriptase. The dye may be any dye of Formula 1 defined above, most preferably 5-Bromo-PAPS or 5-Nitro-PAPS.

In some embodiments, the composition further comprises a metal salt, such as a Mn (II), Mg(II), Ca(II), Fe(II), Re(II), Co(III), Cr(III), Cu(II), Ni(II),or Zn(II) salt; preferably a divalent manganese (Mn²⁺) salt such as MnCl₂, MnSO₄, MnCO₃, or Mn(NO₃)₂. In some embodiments, the composition comprises the dye complexed with a metal ion, preferably a divalent manganese ion (e.g., Mn²⁺), where the metal ion is capable of being removed from the dye-metal complex in the presence of pyrophosphate.

The color of the dyes of Formula 1, more specifically the dyes in FIG. 1B, without the metal ion or after sequestration of the metal ion is yellow. When complexed with the metal ion, the dye has a different color. For example, at the pH of amplification reactions, the Mn²⁺: dye complexes are red and purple-brown for 5-Bromo-PAPS and 5-Nitro-PAPS, respectively. Release of the metal ion (e.g., Mn2+ ion) from the dye results in a color change to yellow.

In some embodiments, the composition may comprise two or more different pyridylazoaniline dyes, preferably wherein at least one is a dye of Formula 1, that produce the same or different visual color in the presence of a divalent metal ion to provide further contrast in colorimetric detection. In other embodiments, two or more divalent metal ions are combined with one or more pyridylazoaniline dyes, preferably dyes of Formula 1, to provide further contrast in colorimetric detection.

FIG. 3A-3C and FIG. 4A-4C illustrate that the concentration of divalent manganese ions was not critical to obtain the desired effect. This was observed at all concentrations of manganese salt tested, namely concentrations in the range of 50 µM -150 µM manganese chloride when combined with 50 µM-100 µM 5-Bromo-PAPS or 5-Nitro-PAPS. Thus, in some embodiments, the composition contains Mn²⁺ at any concentration in the range 10 µM -500 µM, such as in the range 25 µM -250 µM or 50 µM -150 µM; and/or contains the dye at any concentration in the range 10 µM -350 µM, such as in the range 25 µM -150 µM or 50 µM -100 µM. For example, the composition may contain about 25 µM, 50 µM, 75 µM, or 100 µM Mn²⁺ and 50 µM or 75 µM dye; such as 100 µM Mn²⁺ and 75 µM dye, or 50 µM Mn²⁺ and 50 µM dye.

The concentration ratio between a preferred dye and a divalent metal ion in the composition can be varied to provide further contrast in colorimetric detection. Suitable dye to metal ratios were determined for 5-Bromo-PAPS and 5-Nitro-PAPS as shown in FIG. 4A-4C. The ratio of manganese chloride to the dyes in FIG. 1B used in the examples was in the range of 1:2 manganese chloride to dye to 3:1 manganese chloride to dye. However it is expected that this range may be as much as 5:1 to 1:5 of manganese salt to dye. The composition may therefore contain the manganese salt and dye at any ratio in the range of 5:1 to 1:5. In some embodiments, the molar ratio of the manganese salt to dye is greater than 1.

The composition may further comprise a substrate for the enzyme, wherein cleavage of the substrate by the enzyme results in production of pyrophosphate. In some preferred embodiments, dye-metal complexes are not substantially affected by NTPs and any other phosphorylated species that may be present in the composition. The composition may therefore further comprise one or more NTPs selected from rNTPs and dNTPs (e.g., ATP). In some embodiments, the molar ratio of NTPs to manganese salt is greater than 1.

The composition may comprise a nucleic acid sample that contains a target nucleic acid, such as a target DNA or RNA. The nucleic acid (e.g., DNA or RNA target of interest) may be from, for example, a human or animal subject (e.g., human genomic DNA) or from a prokaryote (e.g., plasmid DNA), and/or may comprise nucleic acid from a pathogen such as a bacteria or virus (e.g., viral RNA, such as SARS-CoV-2 RNA). The nucleic acid sample may be a clinical sample selected from the group consisting of: saliva, nasal mucosa, blood, urine, tissue biopsy, and a cell scrape or an environmental sample selected from the group consisting of water, sewerage, soil and plants or animal material for use as food.

The composition may also contain one or more primers.

The composition may further comprise a buffer such as an amplification buffer. As demonstrated herein, PAPS-based detection permits the use of higher volumes of buffering agents, and thus overcomes a critical limitation associated with pH-based colorimetric LAMP.

The composition may further comprise one or more detergent, especially a cationic detergent such as cetylpyridinium chloride (CPC), cetyltrimethylammonium bromide (CTAB), dodecyltrimethylammonium bromide (DTAB), polydiallyldimethylammonium chloride (PDADMAC), or hexadecyltrimethylammonium (CP). The presence of the detergent may enhance the colorimetric dye response; for example, the detergent may enhance the intensity of the signal for samples containing very low amounts of amplified nucleic acid.

The composition may be lyophilized. Alternatively, the composition may be in an aqueous solution.

In some embodiments, the composition is a master mix for a reaction such as an amplification reaction. In other embodiments, the composition may be combined with other reagents so as to form a master mix for a reaction such as an amplification reaction.

### Master mixes

The pyridylazoaniline dye (e.g., preferably of Formula 1) and/or the metal salt (or dye-metal complex) may be combined with one or more reagents in order to detect a reaction that generates pyrophosphates. Examples of such reactions include: amplification of nucleic acids to detect the presence of target nucleic acids; ligation of adaptors in NGS libraries; removal of pyrophosphates from capped RNA for various RNA workflows, measurement of RNA synthesis by RNA polymerase subject to selected promoters etc. The reagents, which may comprise the enzyme that releases pyrophosphate from a substrate, may be incorporated in a master mix for the reaction.

A master mix is a mixture of reagents for adding to a substrate to execute a reaction or series of reactions. The master mix may include all the reagents required for the reaction except for the substrate (e.g., target nucleic acid). Alternatively, a master mix may contain only the essential or 'core' reagents for a reaction but may not include all the reagents used in the reaction. For example, a master mix for an amplification reaction may include a polymerase and dNTPs in a suitable buffer. A master mix for amplification may include a DNA polymerase, dNTPs, and oligonucleotide primers for a specific target. Another master mix may include a reverse transcriptase, DNA polymerase, dNTPs, and optionally oligonucleotide primers. If the master mix is for PCR, a reagent may be included such as an aptamer or antibody to block polymerase activity at room temperature.

Therefore, there is provided a reagent master mix for a reaction, such as an amplification reaction, further comprising a pyridylazoaniline dye (e.g., preferably of Formula 1) and a metal salt (e.g., manganese salt such as Mn²⁺) optionally in the form of a dye:metal complex. There is also provided a combination of: (i) a reagent master mix e.g., for an amplification reaction; and (ii) one or both of a dye of Formula 1 and a metal salt (e.g., manganese salt such as Mn²⁺), such as in the form of a dye:metal complex. Thus, the dye and/or metal salt (or dye:metal complex) may be included within the master mix or may be provided separately for combination with the master mix. Although for workflow convenience, the dye and metal salt (or dye:metal complex) can be contained in an amplification master mix; the dye, metal salt, or complex may alternatively be combined with any single component from the master mix in place of the whole master mix for convenience depending on the design of the assay.

The master mix may further comprise an enzyme that releases pyrophosphate from a substrate, as described above, preferably a DNA or RNA polymerase. The master mix may further comprise a substrate for the enzyme (e.g., one or more NTPs), which releases pyrophosphate when cleaved by the enzyme. Alternatively, the enzyme and/or its substrate may be provided separately for combination with the master mix.

A master mix may include nucleoside triphosphates, primers, adaptors, ligases, nicking endonucleases, restriction endonucleases, DNA glycosylases, and/or exonucleases. Further optional additional reagents in the master mix may include any of a helicase, a nicking endonuclease, guanidine hydrochloride, a detergent, a reducing agent, glycerol, a hygroscopic sugar, a buffer, a uracil DNA glycosylase or dUTP for preventing DNA carryover between reaction samples, and/or magnesium salts where the concentration of magnesium salts should be carefully titrated so as not to inhibit the removal of manganese from the dye in the presence of pyrophosphates while being of sufficient concentration to enable enzyme reactions that require magnesium ions.

In one embodiment, a master mix is provided that is capable of amplifying nucleic acids when combined with a sample containing the nucleic acids. The dye and metal salt (e.g., dye:metal complex) may be included in or combined with any master mix that is designed to accomplish amplification from a target DNA or RNA (in the presence of a reverse transcriptase), such as LAMP, polymerase chain reaction (PCR), strand displacement amplification (SDA/NEAR), helicase dependent amplification (HDA), multiple displacement amplification (MDA), rolling circle amplification (RCA), ligation mediated amplification, whole genome amplification (WGA), nucleic acid sequence-based amplification (NASBA), recombinase polymerase amplification (RPA), Cas-CrispR associated nonspecific amplification (CRISPR-NAS) and transcription mediated amplification (TMA).

An amplification master mix typically includes one or more DNA and/or RNA polymerases. Examples of DNA polymerases include Taq polymerase or archaea polymerase or variants thereof. Variants of archaea polymerases include fusion proteins where the fusion may be between an archaea polymerase and a DNA binding domain that may have specific or non-specific binding properties. The DNA polymerase may be a strand displacing DNA polymerase such as a bacterial polymerase such as Bst or Bsu polymerase where variants include truncations at the N-terminal end or C-terminal end, or mutations there between. Examples of Bst variants are described in US 8,993,298, US 9,127,258, US 9,157,073, EP 2751264, US 9,447,445 and US 9,963,687. DNA polymerases, RNA Polymerases or reverse transcriptases may also be phage polymerases such as Phi 29 DNA polymerase. Optionally, the polymerase is a fusion protein for example, a fusion protein between two different polymerase, between a polymerase and a DNA binding domain and/or binding affinity moiety. The polymerase may be immobilized on a solid substrate such as a bead or column.

The master mix may contain a reverse transcriptase and a DNA polymerase to enable detection of RNA target sequences where RNA is first converted to cDNA by the reverse transcriptase and then amplified by thermocycling (PCR) or isothermal amplification. A master mix may be provided for first strand cDNA synthesis using a reverse transcriptase. A reverse transcriptase and an RNA polymerase may be included in the master mix for NASBA or TMA. Alternatively, a master mix may include an RNA polymerase but not a DNA polymerase or reverse transcriptase for *in vitro* transcription.

Thus, there is provided a master mix comprising a dye of Formula 1 and/ or a metal salt wherein the metal ion forms a complex with the dye (e.g., in the form of a dye:metal complex) in combination with one or more polymerases and/or reverse transcriptases, such as a DNA polymerase. There is also provided a combination of (e.g., kit comprising): (i) a dye of Formula 1 and/or the metal salt (e.g., dye:metal complex); and (ii) a master mix comprising one or more polymerases and/or reverse transcriptases, such as a DNA polymerase.

The master mix may also include NTPs, which may be dNTPs and/or rNTPs. In preferred embodiments, dye-metal complexes are not substantially affected by NTPs and any other phosphorylated species that may be present in the amplification buffer prior to the amplification reaction. The master mix may additionally include one or more of primers, adaptors, ligases, nicking endonucleases, restriction endonucleases, DNA glycosylases and/or exonucleases.

The master mix may contain the reagents at a concentration that is at least 2X, 5X or 10X the concentration required for the reaction, so that the 2X, 5X or 10X master mix can be diluted (2-fold, 5-fold, or 10-fold, respectively) into the sample resulting in a 1X solution.

The master mix may be stored or formulated for storage as a liquid formulation at a cold temperature such as 4°C, -20°C or -70°C, or a temperature therebetween. In these embodiments, the master mix may contain one or more stabilizing agents such as glycerol, such as at least 10% glycerol, preferably more than 20% glycerol such as at least 50% glycerol; and/or one or more detergents, such as one or more nonionic detergents, zwitterionic detergents, anionic detergents, or cationic detergents.

The master mix may alternatively be lyophilized (or formulated for lyophilization) for room temperature storage. In these embodiments, the master mix may contain a suitable sugar for lyophilization such as stachyose, trehalose or other suitable sugar for binding water molecules. In embodiments, the absorbance characteristics of the composition and the pyridylazoaniline dye-divalent metal complex are not substantially affected by the lyophilization process. As shown in FIG. 13, lyophilized master mixes containing a dye of Formula 1 and manganese ions show similar LAMP activity to comparable formulations that are not lyophilized. This represents a significant advantage for extending shelf life or field use of the master mix to enable for example, point of care for infectious disease detection.

It has been demonstrated here that the reagents in the master mix do not substantially affect the sensitivity of the color change of a colorimetric dye following a target specific amplification. In certain circumstances according to the needs of the user, it may be desirable to combine the dye of Formula 1 and metal ion (e.g., dye:metal complex) with a master mix lacking the dye at the time of the reaction.

The dye and metal salt (or dye:metal complex) can be combined with a sample for testing or a control sample. If the dye and metal salt (or dye:metal complex) are provided separately from the master mix or a portion thereof, the master mix or the remaining portion thereof may be added to the sample in a separate step. There is therefore provided a reaction mixture comprising the dye and metal salt (or dye:metal complex), one or more reagents for the reaction (e.g., added to the reaction in the form of a master mix), and a substrate. For example, the reaction mixture may be an amplification reaction mixture comprising the dye and metal salt (or dye:metal complex), a polymerase, one or more NTPs, and a nucleic acid substrate.

### Kits

Kits containing any of the above-described reagents are provided. In one embodiment, there is a provided a kit comprising an enzyme that releases pyrophosphate from a substrate, a pyridylazoaniline dye (preferably a dye of Formula 1), and a metal salt, preferably a divalent manganese salt. The enzyme (and its substrate), dye, and salt are each as described above. For example, a kit may contain an RNA or DNA polymerase, optionally one or more rNTPs or dNTPs, manganese salt, and a dye of Formula 1 as described above. Kits of the invention may also contain any one or more of NTPs, primers, other enzymes, buffer(s), reagents, or reagent compositions; and, in some case, instructions for use.

The components of the kit may be mixed together, separate from one another, or any combination of the two. For example, the kit may comprise a composition of the invention as described above, which comprises the enzyme and the dye, with the metal salt (e.g., manganese salt) provided in the composition or separately. Alternatively, the enzyme may be in one container and the dye and metal salt (e.g., dye:metal complex) may be in a separate container. Alternatively, the enzyme and metal salt may be in one container, and the dye is in a separate container.

In some embodiments, the components of the kit may be combined (or suitable for combination) with each other, and optionally with additional reagents not included in the kit, to form a master mix for a reaction, such as an amplification reaction. In one embodiment, a kit may contain the dye combined with a master mix (e.g., an amplification master mix) as a single reagent. In another embodiment, a kit may contain a container for the dye and a container for a master mix lacking the dye, suitable for their combination in a reaction, such as an amplification reaction.

### Methods for detecting pyrophosphate

There is provided a method for detecting pyrophosphate, comprising:
(a) combining a metal salt, an enzyme, a substrate for the enzyme that releases pyrophosphate when it is cleaved by the enzyme, and a dye of Formula 1 produce a reaction mix; 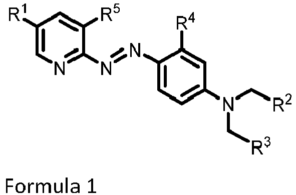 wherein:
   R1 and R5 are each independently selected from the group consisting of H, halogen, nitro, cyano, sulfonic acid, carboxy, trifluoromethyl, trichloromethyl and tribromomethyl;
   R2 and R3 are each independently selected from a lower alkyl optionally comprising a terminal sulfonate group; and
   R4 is H, hydroxyl or carboxyl;
(b) incubating the reaction mix under conditions by which the enzyme cleaves the substrate to produce pyrophosphate; and
(c) observing a change in color of the reaction mix, wherein the change in color indicates production of pyrophosphate. Dyes of Formula 1, metal salts, and enzymes and their substrates for use in the method are each described above.

In some embodiments, the dye is selected from 2-(5-bromo-2-pyridylazo)-5-[N-propyl-N-(3-sulfopropyl)amino]phenol disodium salt dihydrate (5-Bromo-PAPS), 2-(5-nitro-2-pyridylazo)-5-[N-propyl-N-(3-sulfopropyl)amino]phenol disodium salt dihydrate (5-Nitro-PAPS), and 2-(3,5-dibromo-2-pyridylazo)-5-(N-ethyl-N-sulfopropylamino)benzene (3,5-Dibromo-PAESA). In one embodiment, the dye is 2-(5-bromo-2-pyridylazo)-5-[N-propyl-N-(3-sulfopropyl)amino]phenol disodium salt dihydrate (5-Bromo-PAPS), 2-(5-nitro-2-pyridylazo)-5-[N-propyl-N-(3-sulfopropyl)amino]phenol disodium salt dihydrate (5-Nitro-PAPS)

The metal salt contains any metal ion that is (i) capable of forming a complex with the dye through coordination with oxygen of phenolic group and nitrogens of azo and pyridyl groups, and (ii) capable of being removed from the dye-metal complex in the presence of pyrophosphate. The metal salt is preferably a divalent manganese salt (Mn²⁺), such as MnCl₂, MnSO₄, MnCO₃, or Mn(NO₃)₂; preferably MnCl₂. In one embodiment, step (a) of the method comprises combining a complex of the dye and the metal ion (a dye:metal complex) with the enzyme and the substrate to produce the reaction mix. Thus in some embodiments, the dye of Formula 1 is complexed to a metal ion, preferably Mn²⁺ exemplified here.

In one embodiment, step (a) of the method comprises combining the dye and the metal salt (or the dye:metal complex) with the enzyme and optionally further reagents to form a reagent master mix for the reaction, and then combining the master mix with the substrate to produce the reaction mix. Alternatively, step (a) of the method comprises combining the dye and the metal salt (or the dye:metal complex) with the substrate and then adding a pre-formed master mix comprising the enzyme and other reagents for the reaction, to produce the reaction mix.

The enzyme may be any enzyme that cleaves the substrate so as to release pyrophosphate from the substrate, as described herein. For example, the substrate may be an NTP (dNTP and/or rNTP). In preferred embodiments, the dye-metal complex is not substantially affected by NTPs and any other phosphorylated species that may be present in the amplification buffer prior to the amplification reaction. The reaction mix may therefore comprise one or more nucleoside triphosphates (NTPs) selected from rNTPs and dNTPs. Enzymes that hydrolyze dNTPs or rNTPs to release pyrophosphate include for example, DNA polymerase, RNA polymerase, reverse transcriptase, primase, ligase, helicase, nucleotide Ppase/phosphodiesterase, RNA decapping (RNA 5' pyrophosphohydrolase) and geranyl pyrophosphate synthase (used in terpene precursor production). In one embodiment of the method, therefore, the enzyme is a DNA polymerase, an RNA polymerase, or a reverse transcriptase.

The dye and metal salt (or dye:metal complex) may be combined with any one or more of these enzymes and substrate either separately or in combination with one or more other reagents, such as where the reagents are incorporated in a master mix, in order to detect a reaction that generates pyrophosphates. In various enzyme dependent workflows that utilize NTPs, the dye-metal complex can be used as an indicator of NTP hydrolysis. Examples of reactions in which NTP hydrolysis occurs include: amplification of nucleic acids to detect the presence of target nucleic acids; ligation of adaptors in NGS libraries; removal of pyrophosphates from capped RNA for various RNA workflows, measurement of RNA synthesis by RNA polymerase subject to selected promoters etc.

In some embodiments, the metal salt (e.g., a Mn²⁺ salt such as MnCl₂,) is added to the reaction mix at any concentration in the range 10 µM -500 µM, such as in the range 25 µM -250 µM or 50 µM -150 µM; and/or the dye is added to the reaction mix at any concentration in the range 10 µM -350 µM, such as in the range 25 µM -150 µM or 50 µM -100 µM. For example, 25 µM, 50 µM, 75 µM, or 100 µM Mn²⁺ and 50 µM or 75 µM dye; such as 100 µM Mn²⁺ and 75 µM dye, or 50 µM Mn²⁺ and 50 µM dye. Dye to metal salt concentration ratios are discussed above. For example, the metal salt and dye may be added to the reaction mix at any concentration ratio in the range of 5:1 to 1:5 or in the range of 2:1 to 1:2 dye to salt. In some embodiments, the molar ratio of divalent manganese salt to dye is greater than 1.

In one embodiment, the reaction mix comprises the dye, a manganese salt, a polymerase, rNTPs or dNTPs, and a template nucleic acid; the reaction mix is incubated under conditions suitable for amplification of the nucleic acid; and the change in color indicates that a product has been amplified. For example, the reaction mix may comprise an amplification mix for loop-mediated isothermal amplification (LAMP), polymerase chain reaction (PCR), strand displacement amplification (SDA), helicase dependent amplification (HDA), multiple displacement amplification (MDA), rolling circle amplification, ligation mediated amplification, whole genome amplification (WGA), nucleic acid sequence-based amplification (NASBA) or recombinase polymerase amplification (RPA) mix. Conditions suitable for these amplification methods are well-known in the art. For example, if the reaction mix is a PCR reaction mix, step (b) may comprise thermocycling the reaction mix. If the reaction mix is a LAMP reaction mix, step (b) may comprise incubating the reaction mix under isothermal conditions.

As discussed herein, the family of dyes characterized by Formula 1 changes color in the reaction mix as a result of the release of pyrophosphate from the substrate (e.g., by cleavage of NTPs during an amplification reaction) that causes the removal of the metal ions from the dye. In the methods of the invention, a change of color indicates the release of pyrophosphate and hence the cleavage of substrate (whereas the color of a no-substrate control reaction would remain red). The color change may be detected by spectrophotometry, image analysis or by eye. For example, step (c) of the method may comprise detecting a change in hue or a change in peak wavelength. The color change is preferably binary and is the result of an optical density maximum at different distinct wavelengths of light correlated with a positive signal (pyrophosphate production, e.g., amplified nucleic acid) or a negative signal (no pyrophosphate production, e.g., no amplification and therefore no target nucleic acid). In one embodiment, the color change in (c) is from red to yellow, wherein yellow indicates production of pyrophosphate (and hence the cleavage of substrate). The change in color may be detected by observing a change in a ratio of wavelength peak heights, before and after step (b); such as wherein the wavelength peak heights are in the range of 350 nm-620 nm, 430 nm-480 nm and/or 520 nm-620 nm. In one embodiment, the change in color is detected by observing an increase in absorbance at around 450 nm and a decrease in absorbance at around 550 nm.

The dyes and metal salt for use in a non-pH dependent colorimetric assay (e.g., nucleic acid amplification assay) were selected to preferably have two or more, three or more, five or more, or seven or more of the following features:
(a) Minimal adverse effect on the selected reaction (e.g., nucleic acid amplification reaction) when included in the amplification reagent master mix or amplification reaction mix;
(b) Minimal negative effect on sensitivity of the amplification reaction for detecting low amounts of nucleic acid;
(c) Minimal interference with primer binding to target nucleic acid and associated specificity of the reaction;
(d) Readily detectable color change, directly resulting from nucleoside triphosphate hydrolysis;
(e) Distinctly different wavelengths associated with changes in the dye color;
(f) The dye produces a change in visible color in response to changes in concentration of a divalent metal ion (exemplified here by Mn²⁺) that is not substantially affected by the presence of standard buffering agents or a pH between 7 and 9;
(g) The dye is commercially available;
(h) The dye is water soluble;
(i) The dye can be lyophilized.

The sensitivity of the amplification assay for a target nucleic acid is demonstrated herein to be similar in the absence or presence of the dye:metal complex, as exemplified by a pyridylazoaniline dye-manganese complex. It was concluded therefore that the dye:metal complex did not substantially adversely affect the activity of the DNA polymerase or reverse transcriptase.

Colorimetric visual detection according to the method of the invention can thus enable simple detection of pyrophosphate production, for example as a result of nucleic acid amplification, using a visible readout for presence or absence of pyrophosphate and hence the amplification of a DNA or RNA target of interest.

The diagnostic detection of the nucleic acid may require large scale readout of plates in a diagnostic laboratory where the plates contain many samples for testing. In these circumstances, the use of a cheap effective spectrophotometer may be desirable that provides an optical density value for each sample at the selected positive and negative wavelengths where the data can be easily, accurately and cheaply processed by a computer to determine peak ratios or hues from the readout of the spectrophotometer.

Another application of colorimetric detection of amplification is the determination of the concentration of the amplified nucleic acid (e.g., DNA) by determining the ratio of peaks corresponding to the color of the dye:metal complex (e.g., red color) and the uncomplexed dye (e.g., yellow color) after amplification has occurred, and optionally comparing these to peak heights before amplification. In one embodiment, the method of the invention may further comprise determining the amount of template nucleic acid or the amount of amplicon by determining the number of thermocycles in a PCR amplification reaction for producing a predetermined ratio of wavelength peak height.

Determining yield of amplification may be of particular use in NGS library preparation, cloning and synthetic biology. As demonstrated herein, when using the methods of the invention, the yield of PCR amplicon at which a color-change starts to be observable with the naked eye is lower than when using pH-dependent dyes (e.g., at a yield of only 40- 65 ng/µl).

Alternatively, it may be desirable to perform a small numbers of diagnostic tests in the field or at point of care using for example, isothermal amplification. In these circumstances visual detection of target nucleic acid through a clear and unambiguous visual color change is desirable. In this circumstance it may be desirable to photograph the samples and perform image analysis using a cell phone camera or other means to quantify the results.

In workflows where further processing or analysis is conducted on an *in vitro* or *in vivo* synthesized DNA or RNA, a colorimetric analysis ensures success of the amplification step before proceeding with secondary reactions. Examples of such workflows include library preparation for next-generation sequencing, first strand cDNA generation by reverse transcriptase, and *in vitro* transcription by RNA polymerase to produce RNA. An additional use for these colorimetric dyes is for DNA sequencing reactions, where incorporated dNTPs produce a local concentration of pyrophosphate and the corresponding change in color is tied to incorporation of a specific sequence-determined nucleotide.

The recent Covid pandemic has highlighted the need for easy, fast, sensitive, and cost effective viral diagnostic tests for use in large scale (population testing) or small scale (personalized testing) that are capable of detecting viral nucleic acids from body fluids where the most readily accessible are nasal swabs and saliva. Formula 1 dye:metal complexes are here demonstrated to have the attributes required to achieve diagnostic tests such as these. Covid testing also has been used on environmental samples such as wastewater. Again, Formula 1 dye metal complex are a sensitive indicator that can be used with an amplification method of choice for use on site in real time.

Thus, in some embodiments of the method of the invention, the pyridylazoaniline dye, metal salt, and enzyme and its substrate (and other optional reagents as required) are combined with a sample that contains or is suspected to contain a target nucleic acid, which may be a pathogen nucleic acid such as a viral nucleic acid, optionally SARS-CoV-2 RNA; and observing a change of color indicates the presence of the target nucleic acid in the reaction mix. The sample may be any sample described herein, such as a clinical or environmental sample.

Synthetic biology requires creating clones of bacteria containing the synthetic construct(s) in plasmids. Being able to rapidly and easily identify correctly assembled clones can expedite the process of discovery. For example, molecular biology workflows using colony PCR amplification are used to screen for correct assembly of a DNA molecule, and typically require agarose gel electrophoresis. Colorimetric detection of amplification using Formula 1 dye-metal complexes in the reaction mix, as shown here and in the examples is useful for rapid screening of these reactions, avoiding electrophoresis, saving time and increasing throughput.

### EXAMPLES

Below are examples of specific embodiments for carrying out the present invention. The examples are offered for illustrative purposes only and are not intended to limit the scope of the present invention in any way.

### Materials and Methods

Purchased chemical reagents : 5-Bromo- PAPS, 4-(2- pyridylazo)resorcinol), Eriochrome^{®} Black T, MnCl₂, CaCl₂, CuCl₂, ZnCl₂, FeSO₄, NiCl₂, ReCl₃, and CrCl₂, were obtained from Sigma-Aldrich (St. Louis, MO), 5-Nitro-PAPS from Dojindo Molecular Technologies (Rockville, MD), and hydroxynaphthol blue from Acros Organics. All molecular reagents were obtained from New England Biolabs (Ipswich, MA), unless otherwise noted.

For screening metal sensing dyes and metal ions, 25 µL LAMP reactions were performed in triplicate using WarmStart^{®} LAMP Kit (DNA & RNA) (New England Biolabs, Ipswich, MA) with a lambda DNA primer set (Tanner et. al., Biotechniques 58(2), 59-68 (2015)) using 1 ng lambda DNA or NTC. Dyes and metal ions were diluted to 25x concentration and then mixed into LAMP reactions to 1x concentration before incubation. The reactions were incubated at 65°C on a Bio-Rad CFX96^{™} Real-Time PCR instrument (Bio-Rad Laboratories, Hercules, CA) with readings performed at 15 second intervals (108 cycles, ^{~}40 minutes). Before and after incubation, the color of each reaction was recorded by scanning with an office scanner (Epson^{®} Perfection Photo Scanner V600 (Epson, Los Alamitos, CA)). The absorbance of each reaction was measured on a SpectraMax^{®} M5 Microplate Reader (Molecular Devices, San Jose, CA). Thermostable Inorganic Ppase was added (1 Unit) to 25 µL LAMP reactions in the test of pyrophosphate requirement. For comparing tolerance of carryover solutions, the elution buffer (10 mM Tris, pH8.5) from a QIAquick^{®} PCR Purification Kit (Qiagen, Hilden, Germany) and VTM (BD^{™} Universal Viral Transport medium, (Becton Dickinson, Franklin Lakes, NJ)) were used. The pH-based colorimetric LAMP reactions were performed with WarmStart Colorimetric LAMP 2X Master Mix (DNA & RNA) following the product recommendations. For RT- LAMP, the SARS-CoV-2 E1 and N2 primer sets were used along with synthetic SARS-CoV-2 RNA from Twist Bioscience (Twist Synthetic SARS-CoV-2 RNA Control 2) (Twist Bioscience, South San Francisco, CA) diluted to ^{~}10 copies/µL in the presence of 10 ng/µL Jurkat total RNA.

Colorimetric detection of PCR amplification was performed in 25 µL reactions by including 50 µM PAPS dye and 50 µM Mn²⁺ into reactions with either LongAmp Taq 2X Master Mix or OneTaq 2X Master Mix with Standard Buffer. The same concentrations of PAPS dye and Mn²⁺ were added to Q5 High-Fidelity 2X Master Mix and supplemented with Tween 20 to a final concentration 0.1%. The primer pairs for amplifying lambda DNA fragments had the same forward primer 5' CCTGCTCTGCCGCTTCACGC (SEQ ID NO:1) with different reverse primers to create different product lengths. The reverse primers are shown below:
5' TCCGGATAAAAACGTCGATGACATTTGC (SEQ ID NO:2); product length 0.5 kb;
5' GATGACGCATCCTCACGATAATATCCGG (SEQ ID NO:3); product length 1 kb;
5' CCATGATTCAGTGTGCCCGTCTGG (SEQ ID NO:4); product length 2.0 kb; and 5' CGAACGTCGCGCAGAGAAACAGG (SEQ ID NO:5); product length 5.0 kb.

For amplifying 1-2 kb fragments from *E. coli* and 0.5 kb-2 kb human genomic DNA, reactions were performing using LongAmp Hot Start Taq 2X Master Mix with 50 µM Bromo-PAPS, either 25 or 50 µM Mn²⁺, and either 1 ng *E. coli* DNA (^{~}200,000 copies) or 10 ng Jurkat cell DNA (^{∼}2,900 copies).

The primer sequences for the 1 kb *E. coli* fragment are:
Forward: 5' CCTGGATCCAGATGCAGTAATACCGC (SEQ ID NO:6);
Reverse: 5' TCCGAGGATGGTATTCGTCATG (SEQ ID NO:7).
The primer sequences for the 0.5 kb human fragment are:
Forward: 5' GGGGCACCTTCTCCAACTCATACT (SEQ ID NO:8);
Reverse: 5' CGAGCTACCACGCAGACATCAACC (SEQ ID NO:9).

The primer sequences for the 1 kb human fragment are:
Forward: 5' GGGGCACCTTCTCCAACTCATACT (SEQ ID NO:10);
Reverse: 5' CCTCATTTGGGGAGGGGTTATCT (SEQ ID NO:11).

The primer sequences for the 2 kb human fragment are:
Forward: 5' GAAGAGCCAAGGACAGGTAC (SEQ ID NO:12);
Reverse: 5' CCTCCAAATCAAGCCTCTAC (SEQ ID NO:13).

DNA yield for PCR reaction was determined using Quant-iT^{™} PicoGreen^{®} dsDNA Assay Kits (Thermo Fisher Scientific, Waltham, MA).

For colony PCR, a small portion of an *E. coli* colony was transferred using a pipette tip to 15 µL water. 1 µL of the bacterial suspension was used for each PCR reaction with LongAmp.

Similar conditions were used for amplifying the 6,136 bp and 5,292 bp fragments, except that the extension time at 65°C was adjusted to 5 minutes. 5 µL of each PCR product was analyzed by electrophoresis on 1% agarose gel along with a Quick-Load^{®} 1 kb DNA Ladder (New England Biolabs, Ipswich, MA).

The compounds shown in FIG. 1B were tested though data mainly for 5-Bromo-PAPS are provided in the examples in the interest of brevity.

### RESULTS

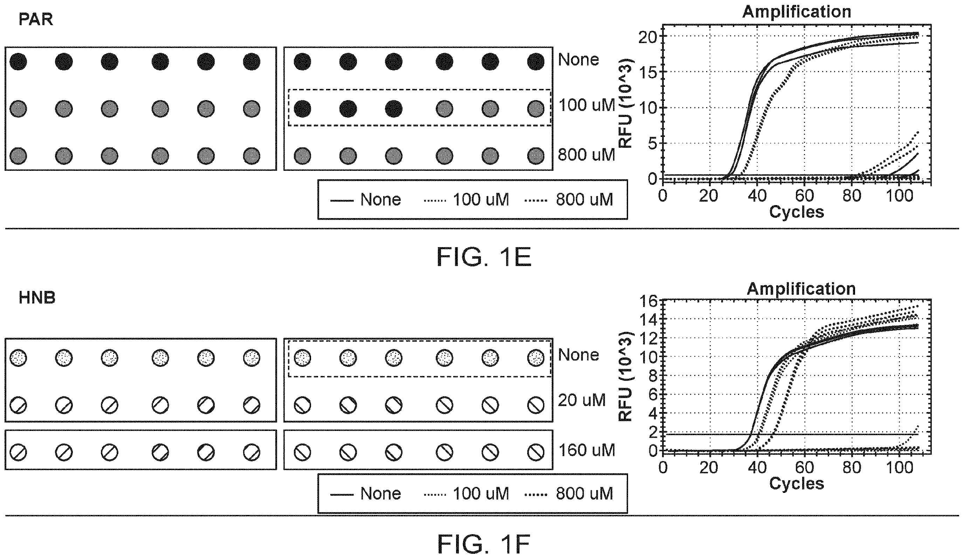

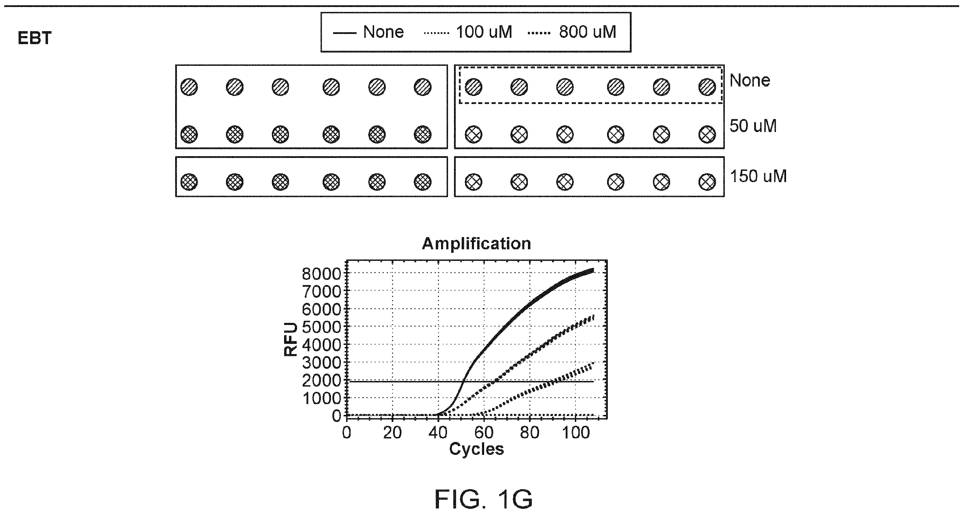

Metal binding dyes in combination with various metal ions were screened in fully buffered (20 mM Tris) reactions. Bright colors were observed with pyridylazoaniline dyes, 5-Bromo-PAPS and 5-Nitro-PAPS before LAMP when complexed with Cu²⁺, Zn²⁺, Fe²⁺ and Ni²⁺ but not with Ca²⁺, Cr²⁺ and Re³⁺. However, the observed colors were substantially unchanged after LAMP except for Mn²⁺ ions. Mn²⁺ changed the color of 5-Bromo-PAPS and 5-Nitro-PAPS from red to yellow at the end of LAMP (FIGs. 2A-2D) and PCR amplification for positive samples (FIG. 8A-8C). A pyridylazo dye 4-(2-pyridylazo)resorcinol (PAR) that had a different core structure also changed color in response to LAMP amplification in the presence of Mn²⁺, but the color contrast was much less pronounced from yellow to orange after amplification (FIG. 1E). Two other dyes, HNB and EBT, which have been used as indicators for LAMP amplification based on Mg²⁺ sensing, had their coloring almost completely quenched by addition of Mn²⁺ (FIG. 1F and 1G).

The hypothesis that the pyridylazoaniline dye complexes with Mn²⁺ to give a red color but is displaced by the Pyrophosphate (PPi) produced during amplification to form insoluble manganese(II) pyrophosphate, thereby restoring the dye original yellow color (FIG. 3C), was tested with 5-Bromo PAPS (FIG. 3A). Inorganic Ppase added to the pyridylazoaniline dye complexed with Mn²⁺ that converts the PPi generated by DNA polymerization to inorganic monophosphate (Pi) abrogated the red-to-yellow color change due to amplification (FIG. 3B). Furthermore, high concentrations of EDTA resulted in red-to-yellow color change immediately, independent of amplification, showing that EDTA chelates the Mn²⁺ more strongly than the pyridylazoaniline dye (FIG. 3B).

Concentration ranges of 5-Bromo-PAPS dyes (50-100 µM) and Mn²⁺ (50 µM -150 µM) were tested to determine whether the ratio was critical. FIGs. 4A-4C show that this was not the case and all ratios tested were effective. Using real-time fluorescence, at these concentrations, neither PAPS dyes nor manganese ions were observed to cause any significant suppression of the LAMP reaction. All combinations provided a high-contrast visual difference between positive and NTC reactions and reliable visual detection of LAMP amplification. 75 µM PAPS dye with 100 µM Mn²⁺ was used in subsequent studies for convenience.

The absorbance spectrum shift of PAPS dyes in completed LAMP reactions is another useful indicator of a positive amplification reaction. In positive reactions, the absorbance at 450 nm significantly increased while the absorption around 550 nm decreased considerably. These shifts of absorbance peaks could be used to quantitatively determine positive reactions in objective automated measurement systems. Two approaches to quantitatively measure positive reactions are shown in FIGs. 5A-5D: using the relative absorbance difference between 450 nm and 550 nm (FIG. 5C) or the ratio between these peaks (FIG. 5D). Both methods provided easily distinguishable readouts of positive and negative reactions. In addition to end point analysis, the absorbance of these two peaks could be measured in real time and applied to determine amplification results, similarly to what was shown for the pH-based colorimetric method used in the high-throughput Color Health COVID-19 colorimetric LAMP assay.

Having determined factors that affect color change with the pyridylazoaniline dyes, the pyridylazoaniline-based detection method was then used to determine the sensitivity of detection of synthetic SARS-CoV-2 RNA using RT-LAMP. 24 reactions were performed with an input of approximately 10 copies of viral RNA per reaction, which is below the limit of detection of a commercially available kit based on a pH-dependent dye (^{~}50 copies, SARS-CoV-2 Rapid Colorimetric LAMP Assay Kit (New England Biolabs, Ipswich, MA). At this low level of nucleic acid, both positive and negative reactions could be detected by eye (FIG. 7A-7D) suggesting that this was the limit of visual detection for this assay. Out of the 24 reactions, 12 showed color change as positive LAMP reaction after a 40 minute incubation. These were also determined to be positive by real-time fluorescence detection. The other 12 reactions showed no color change and matched the 8 NTC reactions which had no amplification as confirmed by real-time detection. By plotting the relative absorption change at 450 nm and 550 nm (FIG.7C) or the ratio between them (FIG. 7D), values for positive reactions consistently showed a large difference from those of negative and NTC reactions, providing an unambiguous identification criterion for virus-specific amplification.

To determine whether pyridylazoaniline dyes and Mn²⁺ affected RT-LAMP detection sensitivity, tests were performed with two primer sets for SARS-CoV-2, with or without guanidine hydrochloride (GuHCI) (Table 1). Each condition was tested with 24 replicates of ^{~}10 copies of SARS-CoV-2 RNA. Comparing the number of positives across the different conditions, 75 µM PAPS dye and 100 µM Mn²⁺, added individually or in combination, had no impact on the detection sensitivity. Moreover, not only was GuHCl compatible with the reaction conditions, but also slightly increased the detection sensitivity.

**Table 1. Sensitivity of RT-LAMP in the presence of 75 µM Bromo-PAPS and/or 100uM Mn²⁺**

| **Primer and GnCl** | **E1700 only** | | **E1700 + 5-Bromo-PAPS** | |
|---|---|---|---|---|
| | No Mn⁺⁺ | 100 uM Mn⁺⁺ | No Mn⁺⁺ | 100 uM Mn⁺⁺ |
| N2 | 6/24 | 5/24 | 4/24 | 7/24 |
| N2 (+GnCI) | 10/24 | 8/24 | 9/24 | 9/24 |
| E1 | 7/24 | 6/24 | 7/24 | 5/24 |
| E1 (+GnCI) | 12/24 | 12/24 | 13/24 | 12/24 |
| N2 + E1 (+GnCI) | 14/24 | 21/24 | 19/24 | 21/24 |

| | | | | |
|---|---|---|---|---|
| ^{∗}The results for reactions with both 5-Bromo-PAPS and Mn⁺⁺ were determined based on color change and the results were identical to that by real time monitoring. | | | | |

Advantages of the pyridylazoaniline colorimetric reporter system over pH- based detection, were demonstrated by testing the tolerance of various buffer carryover (FIG. 6A-6B). For example 0 µL-7 µL (0-28% v/v) of a typical nucleic acid purification column elution buffer (Qiagen EB, 10mM Tris pH 8.5) in 25 µL LAMP reactions with the PAPS reporter system did not interfere with the color change nor inhibit the LAMP reaction. However, with pH-based colorimetric detection, positive and negative reactions displayed much less contrast even with 3 µL -4 µL of the elution buffer. Similarly, 0 µL -7 µL of VTM was tolerated by PAPS-based colorimetric detection, whereas clear visual contrast was only achieved up to 4 µL of VTM with the pH-based system. Tolerance in quantitative measurement systems, e.g., absorbance, is likely even higher than what is clear by eye. These results demonstrate that PAPS-based detection permits the use of higher volumes of buffering agents, and thus overcomes a critical limitation associated with pH-based colorimetric LAMP.

Pyridylazoaniline dyes exemplified by 5-Bromo-PAPS dyes are visual reporters for PCR reactions. 0.5 kb -5.0 kb long fragments from lambda DNA were amplified using PCR in the presence of 50 µM 5-Bromo-PAPS and 50 µM Mn2+ with three commercial PCR master mixes (FIG. 8A) using standard PCR conditions recommended by the manufacturer. Before PCR cycling, all reactions displayed a red color as it was seen in the LAMP mix. After the completion of PCR, reactions containing a template DNA changed color from red to yellow, while the no-template control reactions remained red. Samples with color change matched exactly with those of successful standard PCR amplification, as determined by agarose electrophoresis (FIG. 8B). The results showed that the PAPS dyes were generally compatible with standard PCR mixes. A benefit of PAPS based detection is that an overlay of mineral oil of the samples is not required.

The robustness and sensitivity of PAPS-based colorimetric PCR detection was determined by amplifying DNA fragments from *E.coli* and human genomes for 32-44 cycles and compared the color change with the yield of specific product bands by electrophoresis (FIG. 8A-8D). For a 1.0 kb *E.coli* fragment using 1 ng of input genomic DNA template (^{~}200,000 copies), the reaction color changed to yellow in less than 32 PCR cycles. For a human fragment of the same size using 10 ng of genomic DNA (^{~}2,900 copies), 40 cycles were used to obtain the desired color change.

When the human fragment size increased to 2 kb, the cycle numbers required for the color change reduced to 32-36 cycles. These results indicated that the color change depended on the amount of DNA produced, corroborating the product yields visualized by gel electrophoresis.

The relationship between visual color change and DNA yield was quantified visually and by absorbance spectra (FIG. 10A-10C, FIG. 11A-11C, FIG. 12A-12F). Colorimetric PCR reactions was performed with a range of cycle numbers to amplify low (human genomic DNA, ^{~}2900 copies) and high (lambda DNA, ^{~}1.91x10⁷ copies) copy number templates, including for each 0.5 kb, 1.0 kb and 2.0 kb amplicons. The reactions were checked for visual color change, measured for both absorbance and DNA yield. The absorbance change (A450-A550) indeed correlated with DNA yield, and could be detected at least 4 cycles before the visible color chan. As expected, high copy number of templates for the lambda amplicons required fewer PCR cycles to accumulate similar amount of DNA and change color than that of low copy number human templates. The DNA yield at a point when color change starting to be perceivable by the naked eye were very similar across all amplicons of both lambda and human in the range of 40- 65 ng/ul.

A further example of workflow improvement enabled by colorimetric PCR, can be observed for colony PCR using 5-Bromo-PAPS (FIG. 9A-9D). Colony PCR is routinely used to identify colonies carrying a correct plasmid DNA assembly, directly from a small amount of E. coli cells. It typically requires manual gel electrophoresis to confirm the product presence and size. Cloned plasmids transformed in *E.coli* were chosen for analysis by PCR using PAPS. The best annealing temperature for each primer pair was determined according to routine practice using purified plasmid DNA in the presence of 5-Bromo-PAPS and Mn²⁺ (FIG. 9A). Eight *E.coli* colonies, were analyzed by PCR-four each from two different plasmids. The selected primer pairs were either unique for the first 4 colonies (FIG. 9A and 9B), unique for the second 4 colonies (FIG. 9C), or common for both (FIG. 9D). The resulting PCR reactions changed color only when there was a correct target plasmid and matched perfectly with the specific bands detected by agarose electrophoresis. The PCR results for detecting positive colonies using PAPS was at least as good as obtained with standard agarose gel electrophoresis using a much simpler workflow that avoided gels.

## Claims

1. A composition comprising: (i) an enzyme that releases pyrophosphate from a substrate; and (ii) a dye of Formula 1: 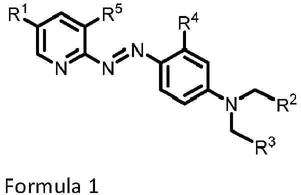 wherein:
R1 and R5 are each independently selected from the group consisting of H, halogen, nitro, cyano, sulfonic acid, carboxyl, trifluoromethyl, trichloromethyl and tribromomethyl;
R2 and R3 are each independently selected from a lower alkyl optionally comprising a terminal sulfonate group; and
R4 is H, hydroxyl or carboxyl.

2. The composition of claim 1, wherein:
at least one of R1 and R5 is F, CI, Br or I; such as wherein R1 is Br;
R2 and R3 are each independently selected from n-butyl, sec-butyl, isobutyl, tert-butyl, propyl, n-propyl, isopropyl, ethyl, or methyl; and at least one of R2 and R3 comprises the terminal sulfonate group.

3. The composition according to claim 1 or claim 2, further comprising a divalent manganese salt (Mn²⁺); optionally wherein the molar ratio of the manganese salt to dye is greater than 1.

4. The composition according to any preceding claim, further comprising a substrate for the enzyme, wherein cleavage of the substrate by the enzyme results in production of pyrophosphate.

5. The composition according to any preceding claim, wherein the composition further comprises one or more nucleoside triphosphates (NTPs) selected from rNTPs and dNTPs; optionally wherein the molar ratio of NTPs to manganese salt is greater than 1.

6. The composition according to any preceding claim, wherein the enzyme is selected from the group consisting of a DNA polymerase, RNA polymerase, reverse transcriptase, primase, ligase, helicase, nucleotide pyrophosphatase (Ppase)/phosphodiesterase, RNA decapping enzyme (RNA 5' pyrophosphohydrolase) and geranyl pyrophosphate synthase; optionally wherein the enzyme is a thermostable DNA polymerase, a strand displacing DNA polymerase, an RNA polymerase or a reverse transcriptase; or wherein the enzyme is a DNA polymerase selected from the group consisting of Bst polymerase, Bsu polymerase, an archaeal DNA polymerase, Taq polymerase, or a variant thereof.

7. The composition according to any preceding claim, wherein the composition is lyophilized or in an aqueous solution.

8. The composition according to any preceding claim, further comprising a nucleic acid sample that contains a target nucleic acid and, optionally, primers; optionally wherein the sample is a sample from an animal subject; or wherein the sample is a clinical sample selected from the group consisting of: saliva, nasal mucosa, blood, urine, tissue biopsy, and a cell scrape or an environmental sample selected from the group consisting of water, sewerage, soil and plants or animal material for use as food.

9. A method for detecting pyrophosphate, comprising:
(a) combining a divalent metal salt, such as a manganese salt; an enzyme; a substrate for the enzyme that releases pyrophosphate when it is cleaved by the enzyme; and a dye of Formula 1 produce a reaction mix; 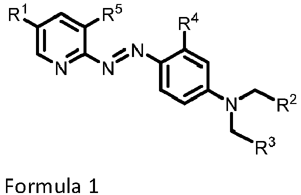 wherein:
R1 and R5 are each independently selected from the group consisting of H, halogen, nitro, cyano, sulfonic acid, carboxyl, trifluoromethyl, trichloromethyl and tribromomethyl;
R2 and R3 are each independently selected from a lower alkyl optionally comprising a terminal sulfonate group; and
R4 is H, hydroxyl or carboxyl;
(b) incubating the reaction mix under conditions by which the enzyme cleaves the substrate to produce pyrophosphate; and
(c) observing a change in color of the reaction mix, wherein the change in color indicates production of pyrophosphate.

10. The method of claim 9, wherein the enzyme is selected from the group consisting of a DNA polymerase, an RNA polymerase, a reverse transcriptase, a primase, a ligase, a helicase, a nucleotide pyrophosphatase (Ppase)/phosphodiesterase, an RNA decapping enzyme (RNA 5' pyrophosphohydrolase) and a geranyl pyrophosphate synthase.

11. The method of claim 9 or 10, wherein the substrate is an NTP.

12. The method of any of claims 9-11, wherein: the reaction mix comprises the manganese salt, the dye, a polymerase, rNTPs or dNTPs, and a template nucleic acid; the reaction mix is incubated under conditions suitable for amplification of the nucleic acid; and the change in color indicates that a product has been amplified.

13. The method of claim 12, wherein the reaction mix comprises an amplification mix for loop-mediated isothermal amplification (LAMP), polymerase chain reaction (PCR), strand displacement amplification (SDA), helicase dependent amplification (HDA), multiple displacement amplification (MDA), rolling circle amplification, ligation mediated amplification, whole genome amplification (WGA), nucleic acid sequence-based amplification (NASBA) or recombinase polymerase amplification (RPA) mix;
optionally wherein the reaction mix is a PCR reaction mix and the method comprises thermocycling the reaction mix; or wherein the reaction mix is a LAMP reaction mix and the method comprises incubating the reaction mix under isothermal conditions.

14. The method according to any of claims 9-13, wherein:
(i) the color change in (c) is red to yellow wherein yellow indicates production of pyrophosphate;
(ii) step (c) comprises detecting the color change by spectrophotometry, image analysis or by eye;
(iii) step (c) comprises detecting the color change by a change in hue or a change in peak wavelength; and/or
(iv) the change in color is detected by observing a change in a ratio of wavelength peak heights, before and after step (b), such as wherein the wavelength peak heights are in the range of 350 nm-620 nm, 430 nm-480 nm and/or 520 nm-620 nm.

15. A kit comprising an enzyme that releases pyrophosphate from a substrate, such as a polymerase, a divalent metal salt, such as a manganese salt; and a dye of Formula 1: 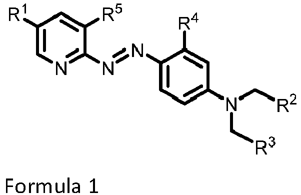 wherein:
R1 and R5 are each independently selected from the group consisting of H, halogen, nitro, cyano, sulfonic acid, carboxyl, trifluoromethyl, trichloromethyl and tribromomethyl
R2 and R3 are each independently selected from a lower alkyl optionally comprising a terminal sulfonate group; and
R4 is H, hydroxyl or carboxyl.

16. The composition, method, or kit according to any preceding claim, wherein the dye is 2-(5-Bromo-2-pyridylazo)-5-(N-propyl-N-sulfopropylamino)phenol (5-Br-PAPS) or 2-(5-Nitro-2-pyridylazo)-5-(N-propyl-N-sulfopropylamino)phenol (5-Nitro-PAPS).
